# EUROPEAN PATENT SPECIFICATION

(11) **EP 3 283 881 B1**
(45) Date of publication and mention of the grant of the patent: **14.12.2022**
(21) Application number: 16715850.0
(22) Date of filing: 12.04.2016
(51) Int. Cl.: G01N 33/543, G01N 1/30, G01N 1/31

(54) **THERMOCHEMICAL-BASED ANTIBODY INACTIVATION METHODS AND SYSTEMS**
VERFAHREN UND SYSTEME ZUR AUF THERMOCHEMIE BASIERTEN ANTIKÖRPERDEAKTIVIERUNG
PROCÉDÉS ET SYSTÈMES D'INACTIVATION D'ANTICORPS À BASE THERMOCHIMIQUE

(30) Priority: 13.04.2015 US 201562146610 P; 25.11.2015 US 201562260147 P
(43) Date of publication of application: 21.02.2018
(73) Proprietor: Ventana Medical Systems, Inc., Tucson, AZ 85755 (US)
(72) Inventor: HUBBARD, Antony, Tucson, Arizona 85719 (US); JONES, Tobin, Tucson, Arizona 85719 (US); TANG, Lei, Oro Valley, Arizona 85737 (US); ZHANG, Wenjun, Tucson, Arizona 85737 (US)
(74) Representative: Altmann Stößel Dick Patentanwälte PartG mbB
(86) International application number: PCT/EP2016/057955
(87) International publication number: WO 2016/166073

(56) References cited:
- WO-A2-2009/117140
- US-B1- 6 296 809
- EDWARD C. STACK ET AL: "Multiplexed immunohistochemistry, imaging, and quantitation: A review, with an assessment of Tyramide signal amplification, multispectral imaging and multiplex analysis", METHODS, vol. 70, no. 1, 1 November 2014 (2014-11-01), pages 46-58, XP055273702, US ISSN: 1046-2023, DOI: 10.1016/j.ymeth.2014.08.016
- TORNEHAVE D ET AL: "Microwaving for double indirect immunofluorescence with primary antibodies from the same species and for staining of mouse tissues with mouse monoclonal antibodies", HISTOCHEMISTRY AND CELL BIOLOGY, SPRINGER, BERLIN, DE, vol. 113, 1 January 2000 (2000-01-01), pages 19-23, XP003012798, ISSN: 0948-6143, DOI: 10.1007/S004180050002
- D. PIRICI ET AL: "Antibody Elution Method for Multiple Immunohistochemistry on Primary Antibodies Raised in the Same Species and of the Same Subtype", JOURNAL OF HISTOCHEMISTRY & CYTOCHEMISTRY, vol. 57, no. 6, 1 June 2009 (2009-06-01), pages 567-575, XP055014242, ISSN: 0022-1554, DOI: 10.1369/jhc.2009.953240

## Description

### FIELD OF THE INVENTION

The present disclosure relates to methods, compositions, and systems for inactivating and/or removing proteinaceous binding entities (e.g., antibodies) for assays such as but not limited to histochemistry assays, more particularly to thermochemical-based treatments and compositions for inactivation and/or removal of proteinaceous binding entities (e.g., antibodies).

### BACKGROUND OF THE INVENTION

Many diagnostic methods require the evaluation of multiple targets in the same sample. Although this is done relatively easily and routinely for molecular techniques, the same cannot be said for microscopic techniques that rely on microscopic visualization. These techniques typically rely on the use of primary/secondary antibody pairs to visualize the target analyte. However, the ability to stain a single sample with multiple primary/secondary antibody pairs is quite limited, due to the potential for cross-reactivity between secondary antibodies and different primary antibodies.

Various methods have been used to help prevent such cross-reactivity in subsequent staining cycles. For example, Nakane (J Histochem Cytochem, 1968, 16:557-60) used an acid wash to remove or elute the antigen-primary antibody-secondary antibody-peroxidase complex from the first staining cycle after the water-insoluble DAB precipitate formed. However, this technique likely caused deterioration in the quality of the tissue sample. Negoescu et al. (J Histochem Cytochem, 1994, 42:433-437) used a secondary polyclonal monovalent F(ab) antibody to block excess binding sites on the primary antibody after the first staining cycle. But, this technique requires carefully controlled experiments and is time-consuming, and the low affinity of the F(ab) secondary antibodies render this approach limiting (Tornehave et al., 2000, Histochem Cell Biol 113:19-23). Wang and Larsson (Histochemistry, 1985, 83:47-56) used formaldehyde vapor treatment to inactivate the antigen-combining sites on the secondary antibodies present in the tissue-bound antigen-antibody complexes. However, this method was associated with the risk of rendering formaldehyde-sensitive antigens undetectable.

Kolodziejczyk and Baertschi (J Histochem Chytochem, 1986, 34:1725-1729) used excessive heat (e.g., 130°C) to remove the immunoglobulins after pictures of a staining were taken. Then a new staining was performed and new pictures were taken and compared to the first set of images. This technique requires several days (e.g., 5-7) of tissue protection in between the different staining cycles, and it can be difficult to predict where pictures should be taken in order to determine co-localization with a subsequent antigen. Lan et al., (J Histochem Cytochem, 1991, 43:97-102) used microwaving to denature already bound antibody molecules to use two primary antibodies and detect nuclear and cytoplasmic antigens using alkaline phosphatase and HRP enzymatic visualization systems. Tomehave et al. (Histochem Cell Biol, 2000, 113:19-23), Toth and Mezey (J Histochem Cytochem, 2007, 55:545), and Osman et al. (Euro J Histochem, 2013, 57:e22) suggested microwaving between the current and subsequent staining cycles to enable double-indirect immunofluorescence staining when the antibodies are same-species. However, this method has not been extensively evaluated for its effectiveness on completely abolishing cross-reactive staining signals, and it was shown that microwaving destroyed some of the antigens in formalin-fixed paraffin embedded (FFPE) samples (Bauer et al., Histochem Cell Biol, 2001, 116:227-232; Osman et al., Euro J Histochem, 2013, 57:e22). The loss of florescence for certain fluorophores was also reported with microwaving techniques (Toth and Mezey, 2007; Bauer et al., 2001). Pirici et al. (J Histochem Cytochem, 2009, 57:567-575) showed microwaving techniques are prone to removing some delicate biopsies from poly-lysine slides, especially if they have already been antigen retrieved by a previous heat-mediated procedure.

Pirici et al. (J Histochem Cytochem, 2009, 57:567-575) and Gendusa et al. (J Histochem Cytochem, 2014, 62:519-531) studied a number of buffers with different pHs, osmolarities, detergents, and denaturing properties in effort to strip the already-bound antibody complex from previous IHC staining cycles. Despite the appeal of the use of buffers for inactivating primary antibodies from previous staining cycles, the buffers were not found to work consistently. For example, Pirici found that a Glycine-HCl/SDS pH2 buffer was effective, but Gendusa found it ineffective. Gendusa found that a 2-mercaptoethanol/SDS pH 6.75 buffer was effective, but Pirici found it ineffective. Also, some buffers were found to decolorize H&E stain or reduce the staining of nuclear protein targets. Other buffers including denaturing agents were determined to be biohazardous.

WO 2009/117140 A2 discloses methods of practicing immunohistochemistry and in situ hybridization analysis, including heating a sample between contacting the sample with a first and a second set of more than one peptide-based probe. Stack et al. (2014), Methods 70(1):46 provides a review on multiplexed immunohistochemistry, imaging, and quantitation. US 6 296 809 B1 teaches an apparatus and methods for automatically staining or treating multiple tissue samples mounted on microscope slides including individualized slide temperature control.

Additionally, none of these methods are compatible with existing automated slide stainers having heat sources for slides, including the Ventana BenchMark and Leica BOND series automated slide stainers. The methods either require specialized reagents that are not currently offered on these automated platforms, or the devices do not contain heat sources capable of reaching the temperatures required. Moreover, none of the commercially available automated slide stainers contain a module capable of heating a slide via microwave.

### SUMMARY OF THE INVENTION

The scope of the invention is defined by the appended claims. Embodiments and descriptions no longer falling under the scope of the appended claims are considered merely as examples suitable for understanding the invention.

Despite the evidence, as described above, that antibody-inactivating buffers were not good candidates for antibody inactivation, inventors have surprisingly discovered a thermochemical process for implementing in between staining cycles so as to help prevent cross-reactivity in subsequent staining cycles. For example, the treatment may help to reduce the amount of an antibody (or antibody-antibody complex, e.g., a primary antibody-secondary antibody complex) in the sample and/or reduce the ability of said antibody (or antibody-antibody complex) to be detected in subsequent staining cycles (e.g., promote elution of the antibody complex, etc.). The sample may be first contacted with (or incubated with) a first exogenous proteinaceous binding entity (PBE), e.g., antibody, in a manner resulting in deposition of the PBE in proximity to its target. The sample may then be contacted with (or incubated with) reagents in a manner resulting in specific deposition of a detectable moiety in proximity to the PBE. Then, the sample may be treated with a thermochemical process/method (heat-kill method) to reduce the ability of the PBE to be further detected in the sample (e.g., to elute the PBE from the sample, to denature the PBE in the sample, etc.).
Thus, the present invention relates to an automated method according to claim 1.

The thermochemical process or method (heat-kill method) of the present invention comprises contacting a sample on a slide with a volume of solution comprising a citrate buffer with a pH from 5 to 7 and heating the slide and sample and solution comprising the buffer via heating a heat source (e.g., heat pad). The heat source may be in close proximity to the slide (e.g., in direct contact or near direct contact with the slide). Heating the heat source effectively heats the slide, sample, and buffer (e.g., raising the temperature from a first temperature to a second temperature). The heat source is heated (e.g., at a particular rate, e.g., a slow rate, e.g., from about 5 to 8 degrees Celsius per minute to about 12 to 15 degrees Celsius per minute) from a starting heat source temperature (e.g., from 18°C to 42°C) to a target heat source temperature (or above the target heat source temperature), e.g., from 80°C to 98°C, wherein the target heat source temperature (or the temperature that is above the target heat source temperature) is less than the boiling point of the solution comprising the buffer. The heat source may be held at the target heat source temperature (or the temperature above the target heat source temperature) for a particular length of time.

The volume of solution that comprises the buffer, which is deposited onto the sample/slide, may be in the range of about 500 µl to about 2 to 4 ml. This volume provides a volume to surface area ratio of 5 µl/cm² to 500 µl/cm² buffer coverage (e.g., the volume of solution that covers that area of the slide). In comparison to bench top methods that feature dunking slides into large volumes of hot buffers, the volume of solution used in the present invention is relatively small. It is widely believed in the industry that a small volume like that of the present invention used when heating a slide and sample to a target sample temperature over a length of time (e.g., at a slow rate) would present problems that would affect the ability to inactivate and/or remove proteinaceous binding entities (e.g., antibodies) as such to make the system ineffective. For example, the heating process may promote too much evaporation of the volume of solution, which would affect the ability to elute unwanted antibody effectively (e.g., antibody could aggregate in a small volume). However, it was surprisingly discovered that a small volume *could* be used in this process to inactivate and/or remove the unwanted proteinaceous binding entities (e.g., antibodies), allowing for detecting at least two targets in the same sample on a single solid substrate.

The present invention features automated methods for detecting at least two targets in the same sample on a single solid substrate. As previously discussed, the method comprises contacting a sample with a first exogenous proteinaceous binding entity (PBE) in a manner resulting in deposition of the PBE in proximity to its target. For example, this may comprise incubating the sample with a primary antibody. The method further comprises contacting the sample with reagents in a manner resulting in specific deposition of a detectable moiety in proximity to the PBE. For example, this may comprise incubating the sample with a secondary antibody specific for the primary antibody (e.g., a secondary antibody specific for the species of the primary antibody, for a tag on the antibody (e.g., a hapten, epitope tag, etc.), etc. The secondary antibody may be labeled, e.g., with a detectable moiety. The secondary antibody/detectable moiety may be deposited in proximity to the PBE).

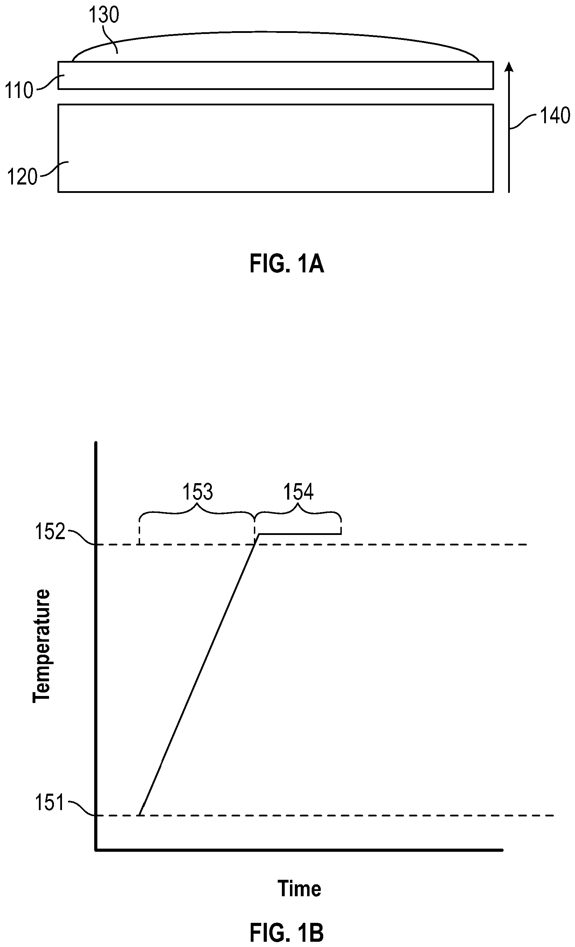

The method further comprises treating the sample to reduce the ability of the first PBE to be further detected in the sample. The sample is contacted with a volume of solution comprising a citrate buffer with a pH from 5 to 7 and the solid substrate is contacted with (or exposed to) a heat source (e.g., heat pad). FIG. 1A shows an example of a solid substrate (e.g., glass slide) with a sample, wherein the solid substrate is atop a heat source (e.g., heat pad). A volume of solution comprising a buffer is atop the sample on the solid substrate. The direction of the heat may be from the heat source to the solid substrate/sample to the solution comprising the buffer.

The buffer comprises a citrate buffer. The solution comprising the buffer is at a starting buffer temperature (which is below the boiling point of the solution comprising the buffer) before the heat source heats the solution comprising the buffer to a second buffer temperature. The heat source is heated from a starting heat source temperature to at least a target heat source temperature (which is below the boiling point of the solution comprising the buffer) over a length of time (see FIG. IB). Without wishing to limit the present invention to any theory or mechanism, it is believed that because the heat source is either in direct contact or in close proximity to the solid substrate (e.g., slide) with the sample, the heat source is capable of heating the slide and sample (and solution comprising the buffer) to a temperature similar to that of the heat source. As previously discussed, heat from the heat source heats the solid substrate/sample, and heat from the solid substrate/sample heats the solution comprising the buffer.

The method further comprises repeating the above steps with a second PBE (and optionally a third PBE, a fourth PBE, a fifth PBE, etc.) and repeating the treatment steps in between incubations with a PBE. The treatment need not be performed after the last PBE incubation. Each detectable moiety is capable of being detected (e.g., visualized) in the sample at the location that corresponds to the PBE's target location. In some embodiments the sample is washed (once, twice, three times, more than three times) after each thermochemical treatment. The method should maintain the quality of the sample such that the sample morphology remains acceptable as determined by a trained reader.

The solution comprises a citrate buffer having a pH from 5 to 7. In some embodiments, the solution comprising the citrate buffer has a pH from 5 to 6.5. In some embodiments, the solution comprising the citrate buffer has a concentration of citrate from 5mM to 20mM. In some embodiments, the solution comprising the citrate buffer further comprises a surfactant. In some embodiments, the surfactant comprises sodium dodecyl sulfate (SDS), sodium lauryl sulfate (SLS), ammonium dodecyl sulfate (ADS), hydrogen dodecyl sulfate (HDS), and tris(hydroxymethyl)aminomethane dodecyl sulfate. In some embodiments, the percentage of surfactant is 0.1%, 0.2%, 0.3%, 0.4%, 0.5%, or 0.6%.

In some embodiments, the volume of solution is a freestanding puddle. The volume of solution covers the sample at a volume to surface area ratio from 5 µl/cm² to 500 µl/cm². In some embodiments, the volume of solution covers the sample at a volume to surface area ratio from 10 µl/cm² to 200 µl/cm². In some embodiments, the volume of solution covers the sample at a volume to surface area ratio from 5 µl/cm² to 100 µl/cm². In some embodiments, the sample is washed (e.g., once, twice, three times, more than three times, etc.) in between the treatment and the addition of a new PBE.

In some embodiments, the starting buffer temperature is from 18°C to 42°C. In some embodiments, the starting buffer temperature is from 20°C to 37°C. In some embodiments, the starting buffer temperature is from 22°C to 30°C. In some embodiments, the heat source comprises a heat pad. In some embodiments, the heat source is in direct contact with the solid substrate. In some embodiments, the starting heat source temperature is from 18°C to 42°C. In some embodiments, the starting heat source temperature is from 20°C to 37°C. In some embodiments, the starting heat source temperature is from 22°C to 30°C. In some embodiments, the starting heat source temperature is from 15°C to 42°C.

In some embodiments, the target heat source temperature is at least 80°C. In some embodiments, the target heat source temperature is 85°C. In some embodiments, the target heat source temperature is 90°C. In some embodiments, the target heat source temperature is 95°C. In some embodiments, the target heat source temperature is 97°C. In some embodiments, the length of time the heat source is heated from the starting heat source temperature to at least the target heat source temperature is at least 3 minutes. In some embodiments, the length of time the heat source is heated from the starting heat source temperature to at least the target heat source temperature is at least 4 minutes. In some embodiments, the length of time the heat source is heated from the starting heat source temperature to at least the target heat source temperature is at least 8 minutes.

In some embodiments, the length of time the heat source is heated from the starting heat source temperature to at least the target heat source temperature is at least 10 minutes. In some embodiments, the length of time the heat source is heated from the starting heat source temperature to at least the target heat source temperature is at least 16 minutes. In some embodiments, the heat source is heated from the starting heat source temperature to at least the target heat source temperature at a rate from 9 to 11 degrees Celsius per minute. In some embodiments, the heat source is heated from the starting heat source temperature to at least the target heat source temperature at a rate from 8 to 12 degrees Celsius per minute. In some embodiments, the heat source is heated from the starting heat source temperature to at least the target heat source temperature at a rate from 8 to 15 degrees Celsius per minute. In some embodiments, the heat source is heated from the starting heat source temperature to at least the target heat source temperature at a rate from 5 to 15 degrees Celsius per minute. In some embodiments, the heat source is at the target heat source temperature or above for at least 15 seconds. In some embodiments, the heat source is at the target heat source temperature or above for at least 30 seconds. In some embodiments, the heat source is at the target heat source temperature or above for at least 1 minute. In some embodiments, the heat source is at the target heat source temperature or above for at least 2 minutes.

In some embodiments, the method comprises incubating the sample with a first primary antibody and incubating the sample with a first secondary antibody specific for the first primary antibody in a manner that deposits a detectable moiety at a site at which the first primary antibody binds to a target; treating the sample to reduce the ability of the primary antibody to be further detected in the sample as described above, repeating the incubation with at least a second primary antibody and a second secondary antibody specific for the second primary antibody; and repeating the treatment in between each repeat of the incubation with primary/secondary antibodies.

In some embodiments, the first primary antibody and the second primary antibody each comprise a tag (e.g., a hapten, epitope tag, etc.). In some embodiments, the tag (e.g., a hapten, epitope tag, etc.) of the first primary antibody and the tag (e.g., a hapten, epitope tag, etc.) of the second primary antibody are the same. In some embodiments, the tag (e.g., a hapten, epitope tag, etc.) of the first primary antibody and the tag (e.g., a hapten, epitope tag, etc.) of the second primary antibody are different. In some embodiments, the second secondary antibody is the same as the first secondary antibody, but the secondary antibodies have different detectable labels.

The method of the present invention is automated. In some embodiments, the method of the present invention is performed in a closed system (e.g., an automated method), wherein the method is free from manual steps. For example, the method is performed in a closed system, and the thermochemical treatment step is integrated into the closed system (the thermochemical treatment is not a manual step).

The present invention also features a system according to claim 13.

The present disclosure also features compositions, not being part of the invention, such as compositions comprising a labeled tissue sample obtained using methods of the present invention. The presentdisclosurealso features compositions comprising a slide with a tissue sample, wherein the tissue sample comprises a primary antibody, a secondary antibody, and a label, wherein the tissue sample is treated as described above.

Without wishing to limit to any theory or mechanism, it is believed that the methods, compositions, and systems described herein may be beneficial for automated assays in automated staining devices (e.g., VENTANA BenchMark, Dako, Leica Bond, etc.). Such automated devices are already adapted to dispense buffers and reagents and to heat samples to various temperatures (e.g., 37°C - 90°C, 37°C - 95°C, 37°C - 99°C, etc.), whereas such automated devices are not adapted to microwave samples or heat samples to very high temperatures such as 130°C.

### BRIEF DESCRIPTION OF THE DRAWINGS

FIG. 1A shows a schematic representation of the solid substrate (110) (e.g., glass slide) with the sample situated atop a heat source (120) (e.g., heat pad). The volume of solution (130) (e.g. puddle), e.g., citrate buffer, is situated atop the sample/solid substrate. Heat from the heat pad is directed upwardly. In some embodiments, the head pad heats the solid substrate. Heat is then transferred upwardly to the sample, then to the volume of solution, e.g., buffer. The volume of solution may be a freestanding puddle. The arrow refers to the direction of heat (140). FIG. 1B is a schematic view of heating of the heat source over time. The heat source starts at a starting heat source temperature (151). The heat source is heated to a target heat source temperature (152) (or above the target heat source temperature) over a length of time. In some embodiments, the heat source is held at the target heat source temperature (or above the target heat source temperature) for a length of time. Number (153) refers to the time from starting heat source temperature to target heat temperature. Number (154) refers to the time at or above the target heat source temperature.

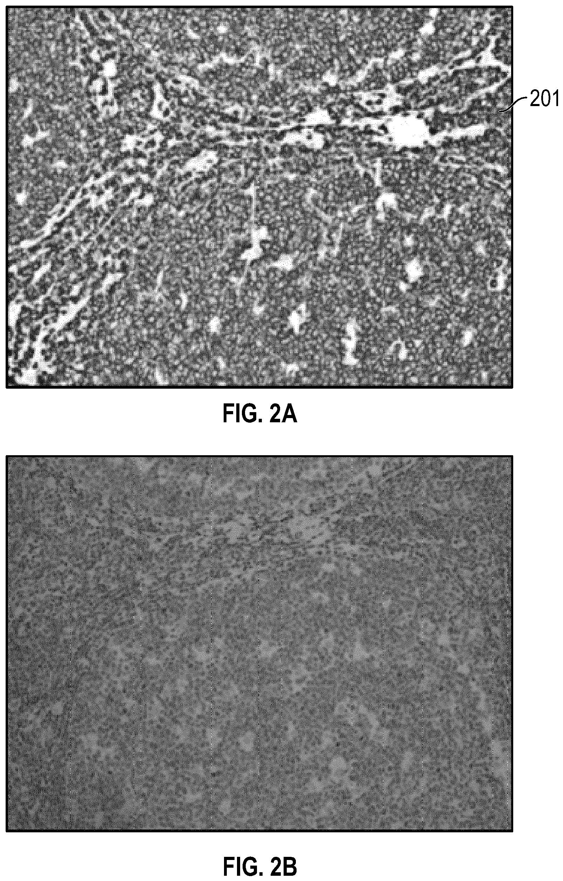

FIG. 2A shows CD20 IHC staining (201) as a control (Example 1). FIG. 2B shows CD20 staining when a thermochemical treatment step (heat-kill step) was used after rabbit-anti-CD20/goat anti-rabbit-HRP application (Example 1).

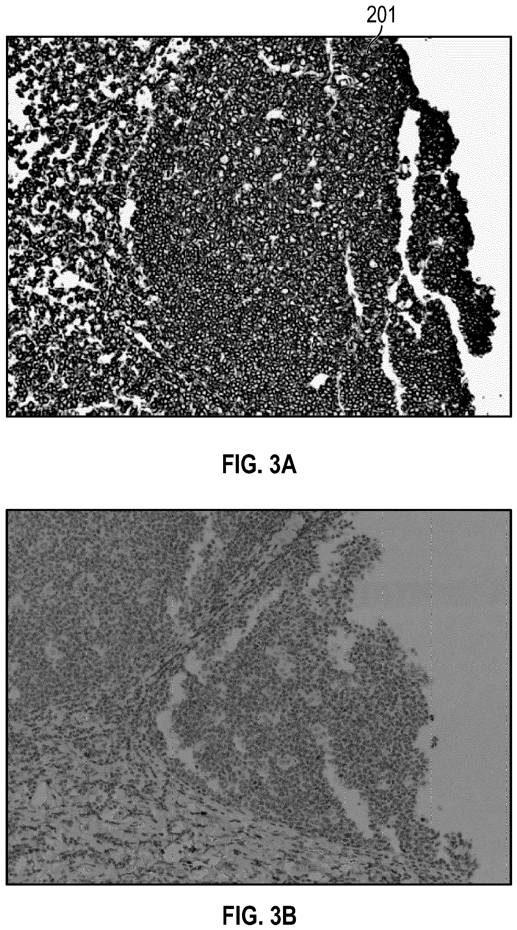

FIG. 3A shows CD20 IHC staining (201) as a control (Example 2). FIG. 3B shows CD20 staining when a thermochemical treatment step (heat-kill step) was applied after rabbit-anti-CD20/goat anti-rabbit-HRP application and DAB detection (Example 2).

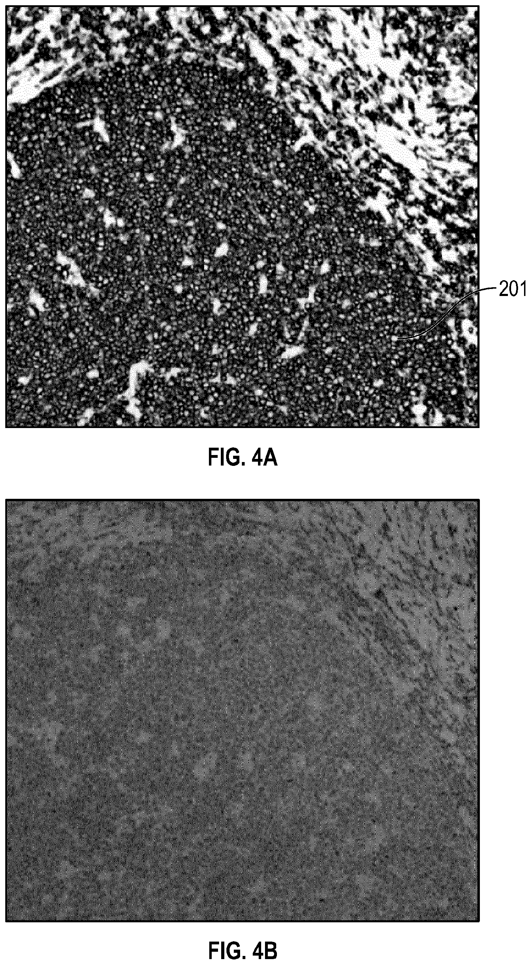

FIG. 4A shows control CD20 IHC staining (201) (Example 3). FIG. 4B shows staining after a thermochemical treatment step (heat-kill step) was applied after the application of the primary and secondary antibodies but before reapplication of secondary antibody (and subsequent DAB detection) (Example 3).

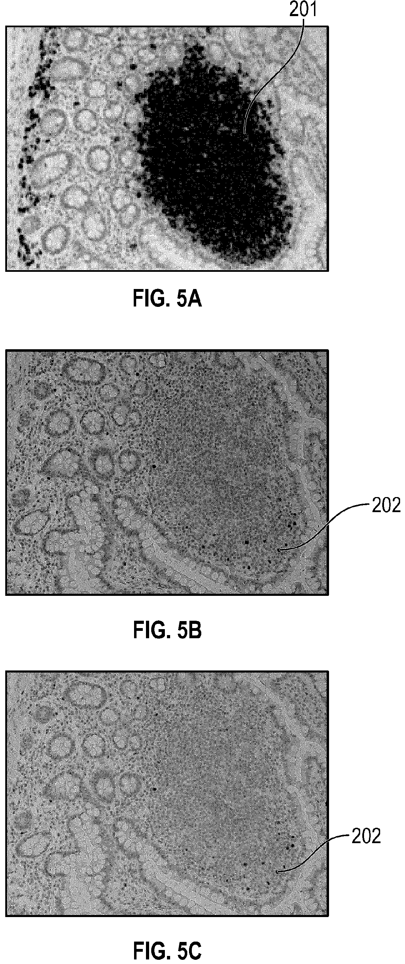

FIG. 5A shows control CD20 staining (201). FIG. 5B shows FoxP3 control staining (202). FIG. 5C shows FoxP3 staining following a thermochemical treatment (heat kill) step after CD20 primary and secondary antibodies were applied but before the FoxP3 primary antibody was applied (Example 4.1).

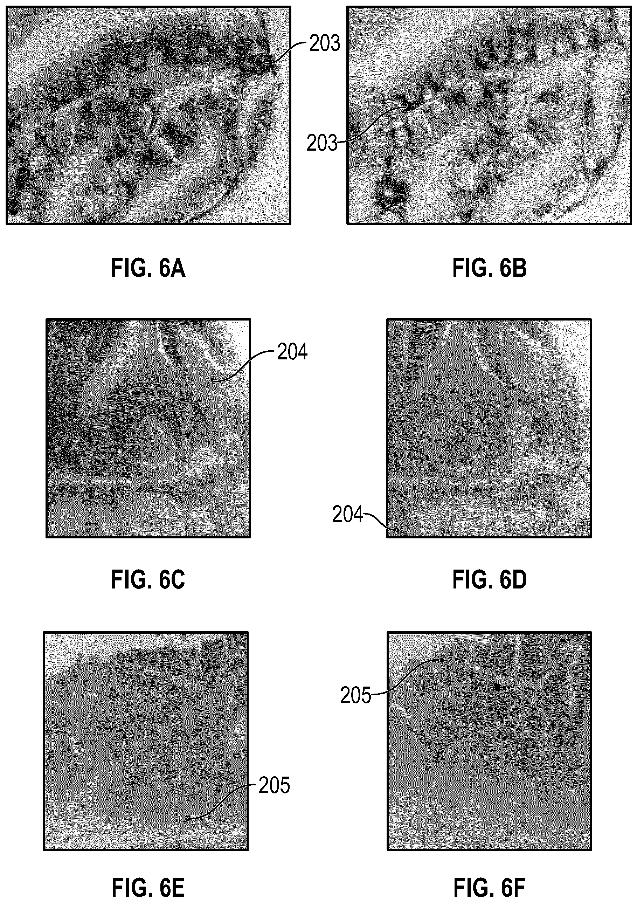

FIG. 6A shows control staining of CD3 (203). FIG. 6B shows CD3 staining after 5 cycles of a heat kill step. CD3 staining was comparable to that of the control. FIG. 6C shows control staining of CD8 (204). FIG. 6D shows CD8 staining after 5 cycles of a heat kill step. CD8 staining was comparable to that of the control. FIG. 6E shows control staining of CD68 (205). FIG. 6F shows CD68 staining after 5 cycles of a heat kill step. CD68 staining was comparable to that of the control (Example 5).

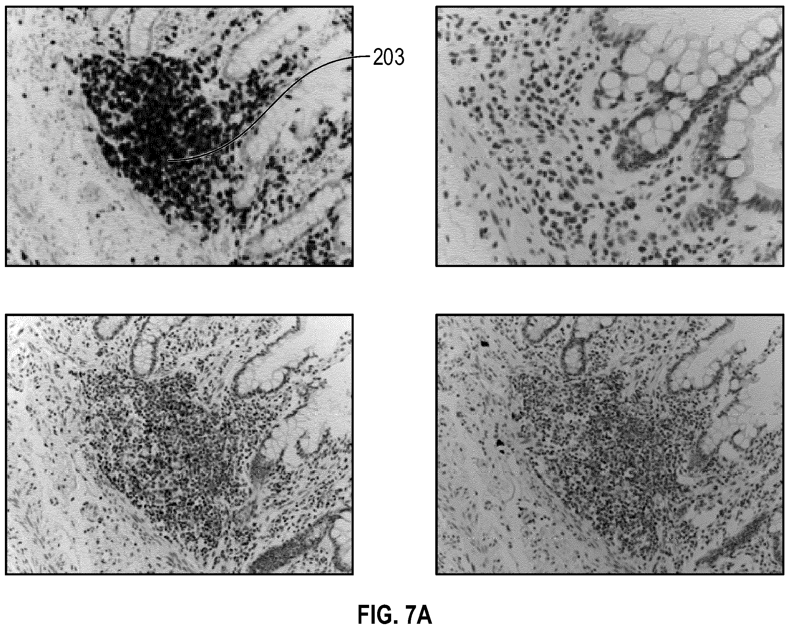

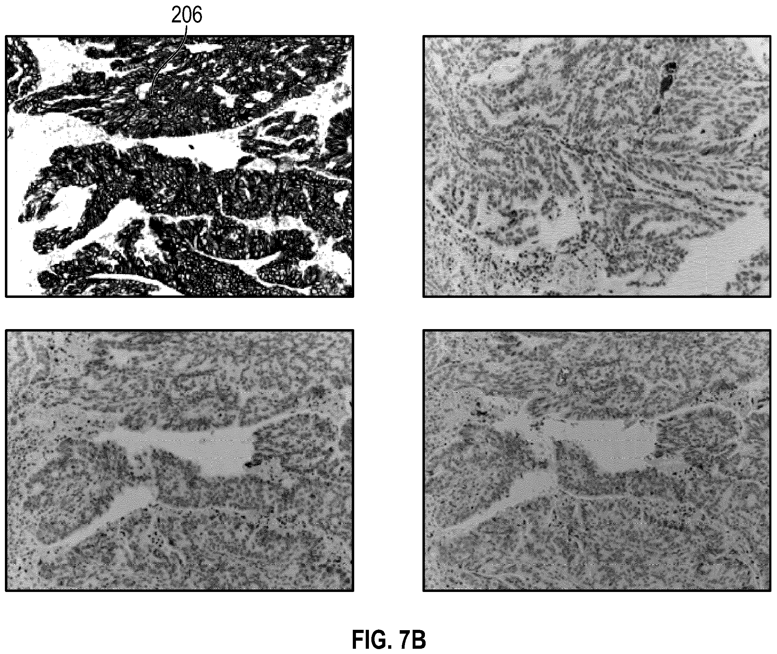

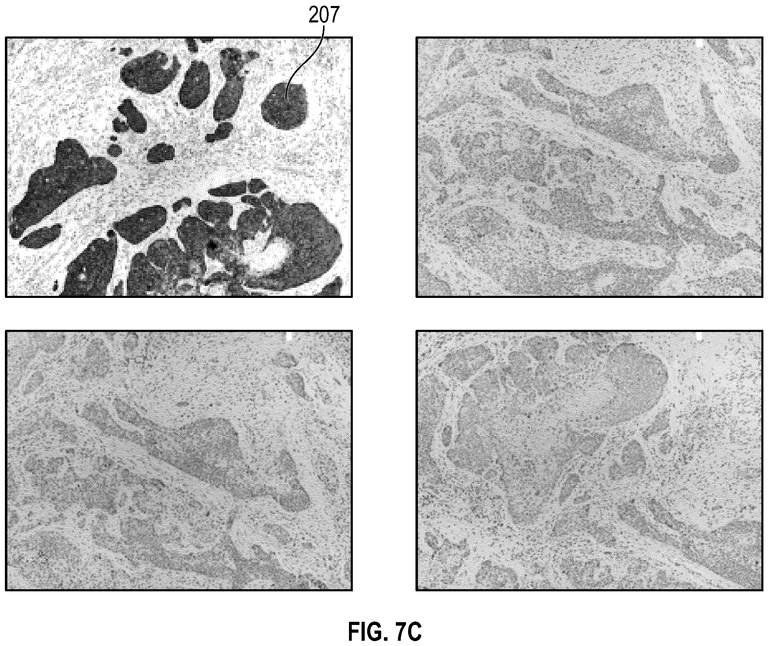

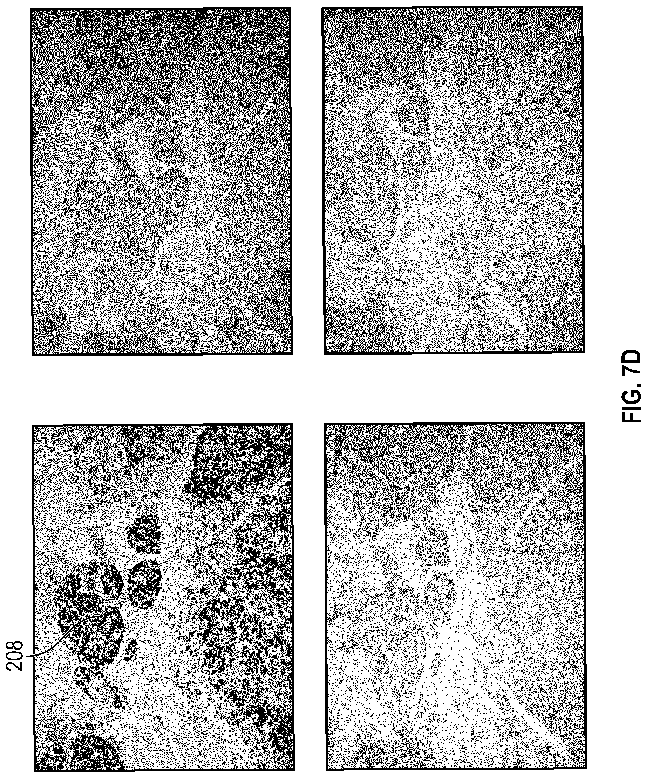

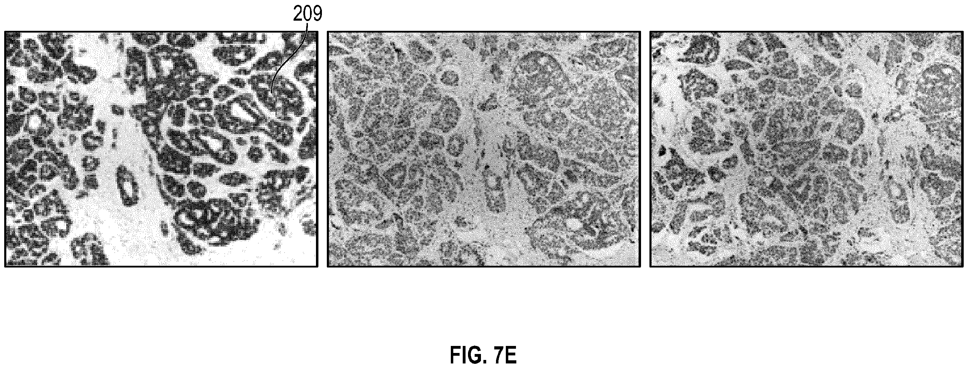

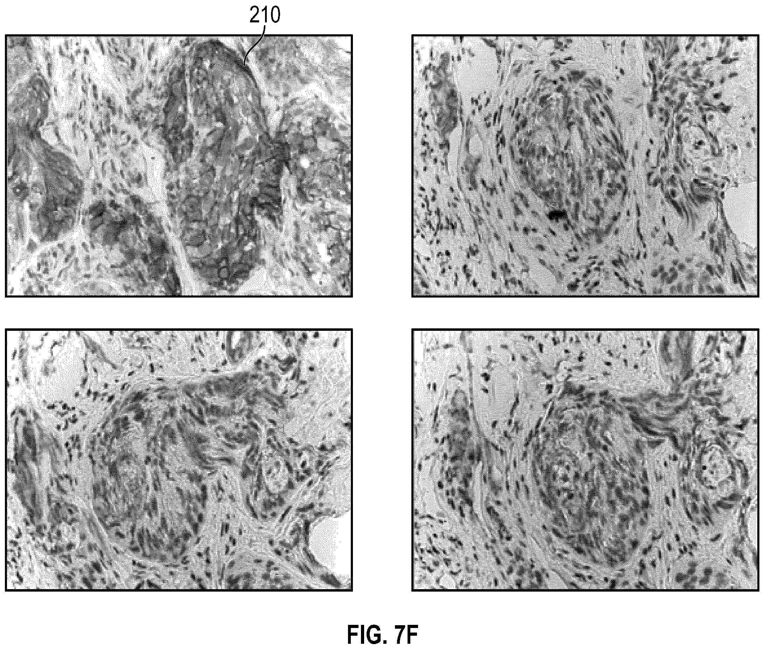

FIG. 7A shows CD3 staining in a colon sample. The top left panel shows control CD3 staining (203). The top right panel shows a control wherein no primary antibody for CD3 was used. The bottom left panel and bottom right panel show the results when a heat-kill step is used. FIG. 7B shows HER2 staining in a breast sample. The top left panel shows control HER2 staining (206). The top right panel shows a control wherein no primary antibody for HER2 was used. The bottom left panel and bottom right panel show the results when a heat-kill step is used. FIG. 7C shows Keratin 5 staining in a head/neck squamous cell carcinoma (HNSCC) sample. The top left panel shows control Keratin 5 staining (207). The top right panel, bottom left panel, and bottom right panel show the results when a heat-kill step is used. FIG. 7D shows Ki-67 staining in a breast sample. The top left panel shows control Ki-67 staining (208). The top right panel shows a control wherein no primary antibody for Ki-67 was used. The bottom left panel and the bottom right panel show the results when a heat-kill step is used. FIG. 7E shows ER staining in a breast sample. The left panel shows control ER staining (209). The middle panel and right panel show the results when a heat-kill step is used. FIG. 7F shows PDL1 staining in a HNSCC sample. The top left panel shows control PDL1 staining (210). The top right panel, the bottom left panel, and the bottom right panel show the results when a heat-kill step is used (Example 8).

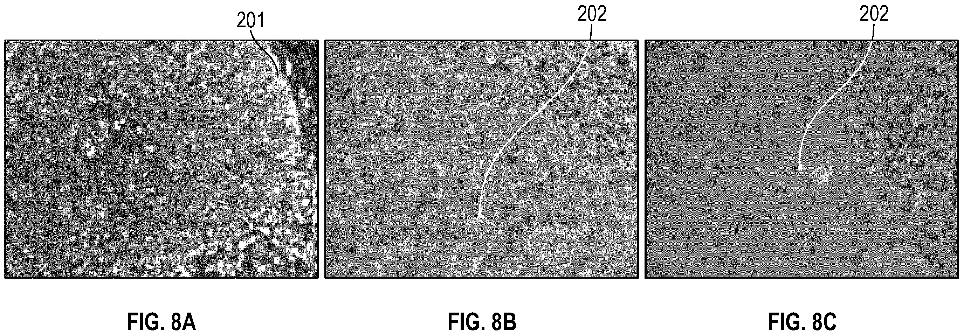

FIG. 8A shows control staining of CD20 (201). FIG. 8B shows control FoxP3 staining (202). FIG. 8C shows FoxP3 staining after a heat-kill step was performed (Example 9).

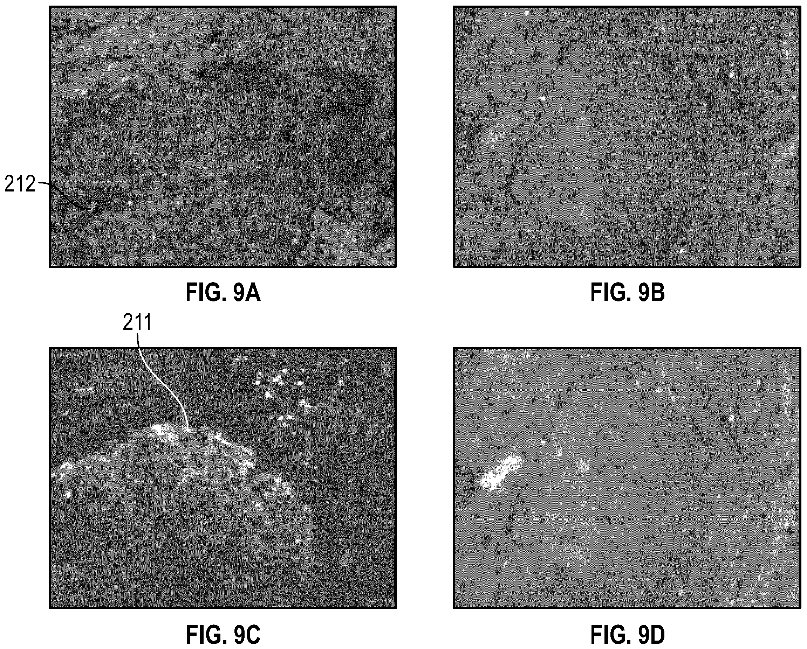

FIG. 9 shows the results of Experiment 3 of Table 1 (see Example 11). FIG. 9A shows DAPI staining (212) (nuclear staining). FIG. 9B shows autofluorescence (DCC) (100 ms exposure). FIG. 9C shows PD-L1 positive staining (211) (another slide) with R6G (staining shows the outlines of the membranes). FIG. 9D shows the PD-L1 staining using R6G following a heat-kill/heat deactivation step (500 ms exposure). Even at a prolonged exposure time of 500 ms, no cross-reactive signal was detected above autofluorescent background level.

### TERMS

Unless otherwise explained, all technical and scientific terms used herein have the same meaning as commonly understood by one of ordinary skill in the art to which a disclosed invention belongs. The singular terms "a," "an," and "the" include plural referents unless context clearly indicates otherwise. Similarly, the word "or" is intended to include "and" unless the context clearly indicates otherwise. "Comprising" means "including." Hence "comprising A or B" means "including A" or "including B" or "including A and B."

Suitable methods and materials for the practice and/or testing of embodiments of the disclosure are described below. Such methods and materials are illustrative only and are not intended to be limiting. Other methods and materials similar or equivalent to those described herein can be used. For example, conventional methods well known in the art to which the disclosure pertains are described in various general and more specific references, including, for example, Sambrook et al., Molecular Cloning: A Laboratory Manual, 2d ed., Cold Spring Harbor Laboratory Press, 1989; Sambrook et al., Molecular Cloning: A Laboratory Manual, 3d ed., Cold Spring Harbor Press, 2001; Ausubel et al., Current Protocols in Molecular Biology, Greene Publishing Associates, 1992 (and Supplements to 2000); Ausubel et al., Short Protocols in Molecular Biology: A Compendium of Methods from Current Protocols in Molecular Biology, 4th ed., Wiley & Sons, 1999; Harlow and Lane, Antibodies: A Laboratory Manual, Cold Spring Harbor Laboratory Press, 1990; and Harlow and Lane, Using Antibodies: A Laboratory Manual, Cold Spring Harbor Laboratory Press, 1999.

Although methods and materials similar or equivalent to those described herein can be used to practice or test the disclosed technology, suitable methods and materials are described below. The materials, methods, and examples are illustrative only and not intended to be limiting.

In order to facilitate review of the various embodiments of the disclosure, the following explanations of specific terms are provided:

**Proteinaceous Binding Entity:** As used herein, the term "binding entity" shall refer to any proteinaceous molecule that is capable of specifically binding to a specific molecular structure. Examples include antibodies and antigen binding fragments thereof, as well as engineered specific binding structures, including ADNECTINs (scaffold based on 10^{th} FN3 fibronectin; Bristol-Myers-Squibb Co.), AFFIBODYs (scaffold based on Z domain of protein A from *S. aureus*; Affibody AB, Solna, Sweden), AVIMERs (scaffold based on domain A/LDL receptor; Amgen, Thousand Oaks, CA), dAbs (scaffold based on VH or VL antibody domain; GlaxoSmithKline PLC, Cambridge, UK), DARPins (scaffold based on Ankyrin repeat proteins; Molecular Partners AG, Zürich, CH), ANTICALINs (scaffold based on lipocalins; Pieris AG, Freising, DE), NANOBODYs (scaffold based on VHH (camelid Ig); Ablynx N/V, Ghent, BE), TRANS-BODYs (scaffold based on Transferrin; Pfizer Inc., New York, NY), SMIPs (Emergent Biosolutions, Inc., Rockville, MD), and TETRANECTINs (scaffold based on C-type lectin domain (CTLD), tetranectin; Borean Pharma A/S, Aarhus, DK). Descriptions of such engineered specific binding structures are reviewed by Wurch et al., Development of Novel Protein Scaffolds as Alternatives to Whole Antibodies for Imaging and Therapy: Status on Discovery Research and Clinical Validation, Current Pharmaceutical Biotechnology, Vol. 9, pp. 502-509 (2008).

As used herein, the term "antibody" refers to any form of antibody that exhibits the desired biological or binding activity. Thus, it is used in the broadest sense and specifically covers, but is not limited to, monoclonal antibodies (including full length monoclonal antibodies), polyclonal antibodies, multispecific antibodies (e.g., bispecific antibodies), humanized antibodies, fully human antibodies, chimeric antibodies and camelized single domain antibodies.

As used herein, unless otherwise indicated, "antibody fragment" or "antigen binding fragment" refers to antigen binding fragments of antibodies, e.g., antibody fragments that retain the ability to bind specifically to the antigen bound by the full-length antibody, e.g., fragments that retain one or more CDR regions. Examples of antibody binding fragments include, but are not limited to, Fab, Fab', F(ab')₂, and Fv fragments; diabodies; linear antibodies; single-chain antibody molecules, e.g., sc-Fv; nanobodies and multispecific antibodies formed from antibody fragments.

A primary antibody may be any of the aforementioned examples of antibodies or binding entities. A secondary antibody is a binding agent that is capable of binding to a primary antibody, e.g., a binding agent that can bind to a tag (e.g., hapten) on the primary antibody, a binding agent that can bind to the primary antibody directly, etc.

**Contacting:** placement that allows association between two or more moieties, particularly direct physical association, for example both in solid form and/or in liquid form (for example, the placement of a biological sample, such as a biological sample affixed to a slide, in contact with a composition, such as a solution containing the probes disclosed herein).

**Detectable label:** A molecule or material that can produce a signal (such as a visual, electrical, or other signal) that indicates the presence and/or amount of a target (such as a protein or nucleic acid) in a sample. Detectable labels are well known to one of ordinary skill in the art.

**Hapten:** A hapten is a molecule, typically a small molecule, which can combine specifically with an antibody, but typically is substantially incapable of being immunogenic except in combination with a carrier molecule. Many haptens are known and frequently used for analytical procedures, such as dinitrophenyl, biotin, digoxigenin, fluorescein, rhodamine, or those disclosed in U.S. Pat. No. 7,695,929.

Other haptens have been specifically developed by Ventana Medical Systems, Inc., assignee of the present application, including haptens selected from oxazoles, pyrazoles, thiazoles, nitroaryls, benzofurans, triterpenes, ureas, thioureas, rotenoids, coumarins, cyclolignans, and combinations thereof, with particular hapten examples of haptens including benzofurazan, nitrophenyl, 4-(2-hydroxyphenyl)-1H-benzo[b][1,4]diazepine-2(3H)-one, and 3-hydroxy-2-quinoxalinecarbamide. Plural different haptens may be coupled to a polymeric carrier. Moreover, compounds, such as haptens, can be coupled to another molecule using a linker, such as an NHS-PEG linker.

**Histochemistry (e.g., immunohistochemistry (IHC)):** A method of determining the presence or distribution of a target molecule in a sample by detecting interaction of the target molecule with a specific binding agent, such as an antibody, that can be detected. For example, a sample is contacted with an antibody under conditions permitting antibody-antigen binding. Antibody-antigen binding can be detected by means of a detectable label conjugated to the antibody (direct detection) or by means of a detectable label conjugated to a secondary antibody, which binds specifically to the primary antibody (e.g., indirect detection).

**Multiplex, -ed, -ing:** Embodiments of the present invention allow multiple targets in the same sample to be detected, e.g., substantially simultaneously, or sequentially, as desired.

**Specific binding:** A term that refers to the binding of an agent that preferentially binds to a defined target (such as a secondary antibody to a primary antibody, an antibody to a specific protein or antigen, etc.). Immunoassay formats can be used to select antibodies that specifically react with a particular protein (such as antibodies that specifically bind HER2 protein or ER protein). See Harlow & Lane, Antibodies, A Laboratory Manual, Cold Spring Harbor Publications, New York (1988), for a description of immunoassay formats and conditions.

**Subject:** Any multi-cellular vertebrate organism, such as human or non-human mammals (*e.g*., veterinary subjects).

**Cellular sample:** Any sample containing intact cells, such as cell cultures, bodily fluid samples or surgical specimens taken for pathological, histological, or cytological interpretation.

**Tissue sample:** A cellular sample that preserves the cross-sectional spatial relationship between the cells as they existed within the subject from which the sample was obtained. "Tissue sample" shall encompass both primary tissue samples (i.e. cells and tissues produced by the subject) and xenografts (i.e. foreign cellular samples implanted into a subject).

**Cytological sample:** A cellular sample in which the cells of the sample have been partially or completely disaggregated, such that the sample no longer reflects the spatial relationship of the cells as they existed in the subject from which the cellular sample was obtained. Examples of cytological samples include tissue scrapings (such as a cervical scraping), fine needle aspirates, samples obtained by lavage of a subject, *et cetera.*

**Stain:** When used as a noun, the term "stain" shall refer to any substance that can be used to visualize specific molecules or structures in a cellular sample for microscopic analysis, including bright field microscopy, fluorescent microscopy, electron microscopy, and the like. When used as a verb, the term "stain" shall refer to any process that results in deposition of a stain on a cellular sample.

### DESCRIPTION OF THE INVENTION

Referring now to FIG. 1-9, the present disclosure features thermochemical (heat-kill) processes, methods, compositions, and systems for helping to prevent cross-reactivity of a detectable protein (e.g., a proteinaceous binding entity (PBE)) in a subsequent staining cycle. The present invention may reduce the amount of the detectable protein and/or renders the detectable protein inactive or undetectable for subsequent staining cycles (e.g., a trained reader would not see much - if any - cross-reactivity). The thermochemical treatment method allows the detectable (e.g., visible) label or marker that is deposited onto the sample subsequent to the incubation of the sample with the detectable protein to remain on the sample (or most of the detectable label remains).

Note that the sample is not limited to a cellular sample such as a tissue sample (e.g., a FFPE tissue sample); in some embodiments, the sample is cell-free. Further, the present invention is not limited to immunohistochemistry applications.

The sample may be first contacted with (or incubated with) a first exogenous proteinaceous binding entity (PBE), e.g., antibody, in a manner resulting in deposition of the PBE in proximity to its target. The sample may then be contacted with (or incubated with) reagents in a manner resulting in specific deposition of a detectable moiety in proximity to the PBE. Then, the sample may be treated with a thermochemical process/method (heat-kill method) to reduce the ability of the PBE to be further detected in the sample (e.g., to elute the PBE from the sample, to denature the PBE in the sample, etc.).

### Thermochemical Treatment

The thermochemical process or method (heat-kill method) of the present invention comprises contacting a sample on a slide with a volume of solution comprising a citrate buffer with a pH from 5 to 7 and heating the slide and sample and solution comprising the buffer via heating a heat source (e.g., heat pad). Heating the heat source effectively heats the slide, sample, and buffer (e.g., raising the temperature from a starting buffer temperature to a second temperature).

FIG. 1A shows a schematic representation of the solid substrate (e.g., glass slide) with the sample situated atop a heat source (e.g., heat pad). The volume of solution with the buffer (e.g. puddle) is situated atop the sample/solid substrate. Heat from the heat source may be directed from the heat source to the solid substrate/sample to the solution comprising the buffer.

In some embodiments, the heat source comprises a heat pad. In some embodiments, the heat source is in direct contact with the solid substrate. In some embodiments, the heat source indirectly contacts the solid substrate. In some embodiments, the heat source is in close proximity to the solid substrate. Without wishing to limit the present invention to any theory or mechanism, it is believed that because the heat source is either in direct contact, in indirect contact, or in close proximity to the solid substrate (e.g., slide) with the sample, the heat source is capable of heating the slide and sample and solution comprising the buffer to a temperature, e.g., a temperature similar to that of the heat source.

In some embodiments, the buffer of the solution comprises citrate. In some embodiments, the solution comprising the buffer has a pH from 5 to 7. In some embodiments, the solution comprising the buffer has a pH from 5 to 6.5. In some embodiments, the solution comprising the buffer has a pH of at least 4.5. In some embodiments, the solution comprising the buffer has a pH of at least 5. In some embodiments, the solution comprising the buffer has a pH of at least 5.5. In some embodiments, the solution comprising the buffer has a pH from 5 to 10, from 5 to 9, from 5 to 8.3, from 5 to 8, from 5 to 7, from 5 to 6.5, from 5.5 to 8, from 5.5 to 7, from 5.5 to 6.5, from 5.8 to 6.3, from 4 to 9, from 4 to 8, from 5 to 6, from 4 to 7.5, from 5.5 to 6.2, from 5.9 to 6.9, etc.

In some embodiments, the solution comprising the buffer has a concentration of citrate from 5mM to 20mM. In some embodiments, the solution comprising the buffer has a concentration of citrate from 1mM to 50mM. In some embodiments, the solution comprising the buffer has a concentration of citrate of 8mM, 10mM, 12mM, 14mM, 15mM, 18mM, etc. In some embodiments, the buffer comprises citrate and acetate. In some embodiments, the buffer is citrate and acetate, and the citrate is at a concentration of 5mM to 20mM. The buffer may be one or more selected from the group consisting of acetate, MES, citrate, citrate phosphate, phosphate, tris, borate, bicarbonate/carbonate, and carbonate buffers.

In some embodiments, the solution comprising the buffer comprises a chelating agent, e.g., citrate, EGTA, EDTA, phosphonate, etc. In some embodiments, the solution comprising the buffer further comprises a surfactant. Surfactants may include but are not limited to sodium dodecyl sulfate (SDS), sodium lauryl sulfate (SLS), ammonium dodecyl sulfate (ADS), hydrogen dodecyl sulfate (HDS), and tris(hydroxymethyl) aminomethane dodecyl sulfate, the like, or a combination thereof. In some embodiments, the percentage of surfactant is 0.1%, 0.2%, 0.3%, 0.4%, 0.5%, 0.6%, from 0.01% to 5%, from 0.05% to 3%, from 0.1% to 1%, etc. In some embodiments, the solution comprising the buffer further comprises a detergent, e.g., Colaterge LFD-C. In some embodiments, the percentage of detergent (e.g., w/v) is from 1% to 15%, e.g., 3%, 4%, 5%, 6%, 7%, 8%, 9%, 10%, from 1% to 3%, etc. The present invention is not limited to the aforementioned surfactants, buffers, and concentrations thereof.

The volume of solution that comprises the buffer, which is deposited onto the solid substrate (e.g., all or a portion of the solid substrate, e.g., atop the sample), may be in the range of about 500 µl to about 2 to 4 ml. This volume provides a volume to surface area ratio, wherein a certain volume of solution covers a certain area of the solid substrate (e.g., slide). For example, in some embodiments, the solution provides a volume to surface area ratio from 5 µl/cm² to 500 µl/cm² coverage over either all or a portion of the solid substrate (e.g., slide), e.g., from 5 µl to 500 µl covers each square centimeter of the solid substrate (or the area of the sample on the solid substrate). The volume of solution may be a freestanding puddle. In some embodiments, the volume of solution covers the sample at a volume to surface area ratio from 5 µl/cm² to 500 µl/cm². In some embodiments, the volume of solution covers the sample at a volume to surface area ratio from 10 µl/cm² to 200 µl/cm². In some embodiments, the volume of solution covers the sample at a volume to surface area ratio from 5 µl/cm² to 100 µl/cm².

### Heating of Heat Source and Solution Comprising Buffer

The heat source is heated from a starting heat source temperature to a target heat source temperature or above over a length of time. The heating of the heat source results in the raising of the temperature of the solution comprising the buffer from a starting buffer temperature to a second temperature. The starting heat source temperature, the target heat source temperature, the starting buffer temperature, and/or the second temperature may each be below the boiling point of the solution comprising the buffer. FIG. 1B shows a schematic of the heating of the heat source over time. Without wishing to limit the present invention to any theory or mechanism, it is believed that because the heat source is either in direct contact or in close proximity to the slide with the sample, the heat source is capable of heating the slide and sample (and buffer) to a temperature similar to that of the heat source.

The heat source is heated (e.g., at a particular rate, e.g., a slow rate, e.g., from about 5 to 8 degrees Celsius per minute to about 12 to 15 degrees Celsius per minute) from a starting heat source temperature (e.g., from 18°C to 42°C) to a target heat source temperature (or above the target heat source temperature), e.g., from 80°C to 98°C, wherein the target heat source temperature (or the temperature that is above the target heat source temperature) is less than the boiling point of the solution comprising the buffer. The heat source may be held at the target heat source temperature (or the temperature above the target heat source temperature) for a particular length of time.

In some embodiments, the starting buffer temperature is less than 50°C. In some embodiments, the starting buffer temperature is less than 45°C. In some embodiments, the starting buffer temperature is less than 42°C. In some embodiments, the starting buffer temperature is less than 40°C. In some embodiments, the starting buffer temperature is less than 37°C. In some embodiments, the starting buffer temperature is less than 35°C. In some embodiments, the starting buffer temperature is less than 32°C. In some embodiments, the starting buffer temperature is less than 30°C. In some embodiments, the starting buffer temperature is less than 28°C. In some embodiments, the starting buffer temperature is less than 25°C. In some embodiments, the starting buffer temperature is less than 22°C. In some embodiments, the starting buffer temperature is from 18°C to 42°C. In some embodiments, the starting buffer temperature is from 20°C to 37°C. In some embodiments, the starting buffer temperature is from 22°C to 30°C. In some embodiments, the starting buffer temperature is from 15°C to 42°C. In some embodiments, the starting buffer temperature is 20°C. In some embodiments, the starting buffer temperature is 25°C. In some embodiments, the starting buffer temperature is 30°C. In some embodiments, the starting buffer temperature is 32°C. In some embodiments, the starting buffer temperature is 37°C. In some embodiments, the starting buffer temperature is 40°C. In some embodiments, the starting buffer temperature is 45°C.

In some embodiments, the starting heat source temperature is less than 50°C. In some embodiments, the starting heat source temperature is less than 45°C. In some embodiments, the starting heat source temperature is less than 42°C. In some embodiments, the starting heat source temperature is less than 40°C. In some embodiments, the starting heat source temperature is less than 37°C. In some embodiments, the starting heat source temperature is less than 35°C. In some embodiments, the starting heat source temperature is less than 32°C. In some embodiments, the starting heat source temperature is less than 30°C. In some embodiments, the starting heat source temperature is less than 28°C. In some embodiments, the starting heat source temperature is less than 25°C. In some embodiments, the starting heat source temperature is less than 22°C. In some embodiments, the starting heat source temperature is from 18°C to 42°C. In some embodiments, the starting heat source temperature is from 20°C to 37°C. In some embodiments, the starting heat source temperature is from 22°C to 30°C. In some embodiments, the starting heat source temperature is from 15°C to 42°C. In some embodiments, the starting heat source temperature is 20°C. In some embodiments, the starting heat source temperature is 25°C. In some embodiments, the starting heat source temperature is 30°C. In some embodiments, the starting heat source temperature is 32°C. In some embodiments, the starting heat source temperature is 37°C. In some embodiments, the starting heat source temperature is 40°C. In some embodiments, the starting heat source temperature is 45°C.

In some embodiments, the target heat source temperature is 75°C. In some embodiments, the target heat source temperature is 76°C. In some embodiments, the target heat source temperature is 77°C. In some embodiments, the target heat source temperature is 78°C. In some embodiments, the target heat source temperature is 79°C. In some embodiments, the target heat source temperature is 80°C. In some embodiments, the target heat source temperature is 81°C. In some embodiments, the target heat source temperature is 82°C. In some embodiments, the target heat source temperature is 83°C. In some embodiments, the target heat source temperature is 84°C. In some embodiments, the target heat source temperature is 85°C. In some embodiments, the target heat source temperature is 86°C. In some embodiments, the target heat source temperature is 87°C. In some embodiments, the target heat source temperature is 88°C. In some embodiments, the target heat source temperature is 89°C. In some embodiments, the target heat source temperature is 90°C. In some embodiments, the target heat source temperature is 91°C. In some embodiments, the target heat source temperature is 92°C. In some embodiments, the target heat source temperature is 93°C. In some embodiments, the target heat source temperature is 94°C. In some embodiments, the target heat source temperature is 95°C. In some embodiments, the target heat source temperature is 96°C. In some embodiments, the target heat source temperature is 97°C. In some embodiments, the target heat source temperature is 98°C. In some embodiments, the target heat source temperature is 99°C. In some embodiments, the target heat source temperature is 100°C. In some embodiments, the target heat source temperature is from 75°C to 100°C. In some embodiments, the target heat source temperature is from 80°C to 100°C. In some embodiments, the target heat source temperature is from 80°C to 98°C. In some embodiments, the target heat source temperature is from 85°C to 100°C. In some embodiments, the target heat source temperature is from 85°C to 98°C. In some embodiments, the target heat source temperature is from 85°C to 95°C. In some embodiments, the target heat source temperature is at least 75°C. In some embodiments, the target heat source temperature is at least 80°C. In some embodiments, the target heat source temperature is at least 85°C. In some embodiments, the target heat source temperature is at least 90°C. In some embodiments, the target heat source temperature is at least 95°C.

In some embodiments, the second temperature of the solution comprising the buffer is the same as the target heat source temperature. In some embodiments, the second temperature of the solution comprising the buffer is 75°C. In some embodiments, the second temperature of the solution comprising the buffer is 76°C. In some embodiments, the second temperature of the solution comprising the buffer is 77°C. In some embodiments, the second temperature of the solution comprising the buffer is 78°C. In some embodiments, the second temperature of the solution comprising the buffer is 79°C. In some embodiments, the second temperature of the solution comprising the buffer is 80°C. In some embodiments, the second temperature of the solution comprising the buffer is 81°C. In some embodiments, the second temperature of the solution comprising the buffer is 82°C. In some embodiments, the second temperature of the solution comprising the buffer is 83°C. In some embodiments, the second temperature of the solution comprising the buffer is 84°C. In some embodiments, the second temperature of the solution comprising the buffer is 85°C. In some embodiments, the second temperature of the solution comprising the buffer is 86°C. In some embodiments, the second temperature of the solution comprising the buffer is 87°C. In some embodiments, the second temperature of the solution comprising the buffer is 88°C. In some embodiments, the second temperature of the solution comprising the buffer is 89°C. In some embodiments, the second temperature of the solution comprising the buffer is 90°C. In some embodiments, the second temperature of the solution comprising the buffer is 91°C. In some embodiments, the second temperature of the solution comprising the buffer is 92°C. In some embodiments, the second temperature of the solution comprising the buffer is 93°C. In some embodiments, the second temperature of the solution comprising the buffer is 94°C. In some embodiments, the second temperature of the solution comprising the buffer is 95°C. In some embodiments, the second temperature of the solution comprising the buffer is 96°C. In some embodiments, the second temperature of the solution comprising the buffer is 97°C. In some embodiments, the second temperature of the solution comprising the buffer is 98°C. In some embodiments, the second temperature of the solution comprising the buffer is 99°C. In some embodiments, the second temperature of the solution comprising the buffer is 100°C. In some embodiments, the second temperature of the solution comprising the buffer is from 75°C to 100°C. In some embodiments, the second temperature of the solution comprising the buffer is from 80°C to 100°C. In some embodiments, the second temperature of the solution comprising the buffer is from 80°C to 98°C. In some embodiments, the second temperature of the solution comprising the buffer is from 85°C to 100°C. In some embodiments, the second temperature of the solution comprising the buffer is from 85°C to 98°C. In some embodiments, the second temperature of the solution comprising the buffer is from 85°C to 95°C. In some embodiments, the second temperature of the solution comprising the buffer is at least 75°C. In some embodiments, the second temperature of the solution comprising the buffer is at least 80°C. In some embodiments, the second temperature of the solution comprising the buffer is at least 85°C. In some embodiments, the second temperature of the solution comprising the buffer is at least 90°C. In some embodiments, the second temperature of the solution comprising the buffer is at least 95°C.

In some embodiments, the length of time the heat source is heated from the starting heat source temperature to the target heat source temperature or above is at least 1 minute. In some embodiments, the length of time the heat source is heated from the starting heat source temperature to the target heat source temperature or above is at least 2 minutes. In some embodiments, the length of time the heat source is heated from the starting heat source temperature to the target heat source temperature or above is at least 3 minutes. In some embodiments, the length of time the heat source is heated from the starting heat source temperature to the target heat source temperature or above is at least 4 minutes. In some embodiments, the length of time the heat source is heated from the starting heat source temperature to the target heat source temperature or above is at least 5 minutes. In some embodiments, the length of time the heat source is heated from the starting heat source temperature to the target heat source temperature or above is at least 6 minutes. In some embodiments, the length of time the heat source is heated from the starting heat source temperature to the target heat source temperature or above is at least 7 minutes. In some embodiments, the length of time the heat source is heated from the starting heat source temperature to the target heat source temperature or above is at least 8 minutes. In some embodiments, the length of time the heat source is heated from the starting heat source temperature to the target heat source temperature or above is at least 9 minutes. In some embodiments, the length of time the heat source is heated from the starting heat source temperature to the target heat source temperature or above is at least 10 minutes. In some embodiments, the length of time the heat source is heated from the starting heat source temperature to the target heat source temperature or above is at least 12 minutes. In some embodiments, the length of time the heat source is heated from the starting heat source temperature to the target heat source temperature or above is at least 14 minutes. In some embodiments, the length of time the heat source is heated from the starting heat source temperature to the target heat source temperature or above is at least 16 minutes. In some embodiments, the length of time the heat source is heated from the starting heat source temperature to the target heat source temperature or above is at least 18 minutes. In some embodiments, the length of time the heat source is heated from the starting heat source temperature to the target heat source temperature or above is at least 20 minutes. In some embodiments, the length of time the heat source is heated from the starting heat source temperature to the target heat source temperature or above is from 2 to 20 minutes. In some embodiments, the length of time the heat source is heated from the starting heat source temperature to the target heat source temperature or above is from 4 to 20 minutes. In some embodiments, the length of time the heat source is heated from the starting heat source temperature to the target heat source temperature or above is from 8 to 20 minutes. In some embodiments, the length of time the heat source is heated from the starting heat source temperature to the target heat source temperature or above is from 8 to 16 minutes.

In some embodiments, the heat source is heated from the starting heat source temperature to at least the target heat source temperature at a rate from 9 to 11 degrees Celsius per minute. In some embodiments, the heat source is heated from the starting heat source temperature to at least the target heat source temperature at a rate from 8 to 12 degrees Celsius per minute. In some embodiments, the heat source is heated from the starting heat source temperature to at least the target heat source temperature at a rate from 8 to 15 degrees Celsius per minute. In some embodiments, the heat source is heated from the starting heat source temperature to at least the target heat source temperature at a rate from 5 to 15 degrees Celsius per minute. In some embodiments, the heat source is heated from the starting heat source temperature to at least the target heat source temperature at a rate from 10 to 15 degrees Celsius per minute. In some embodiments, the heat source is heated from the starting heat source temperature to at least the target heat source temperature at a rate from 5 to 20 degrees Celsius per minute. In some embodiments, the heat source is heated from the starting heat source temperature to at least the target heat source temperature at a rate from 8 to 20 degrees Celsius per minute. In some embodiments, the heat source is heated from the starting heat source temperature to at least the target heat source temperature at a rate of at least 5 degrees Celsius per minute. In some embodiments, the heat source is heated from the starting heat source temperature to at least the target heat source temperature at a rate of at least 8 degrees Celsius per minute. In some embodiments, the heat source is heated from the starting heat source temperature to at least the target heat source temperature at a rate of at least 10 degrees Celsius per minute. In some embodiments, the heat source is heated from the starting heat source temperature to at least the target heat source temperature at a rate of less than 20 degrees Celsius per minute. In some embodiments, the heat source is heated from the starting heat source temperature to at least the target heat source temperature at a rate of less than 15 degrees Celsius per minute. In some embodiments, the heat source is heated from the starting heat source temperature to at least the target heat source temperature at a rate of less than 12 degrees Celsius per minute. In some embodiments, the heat source is heated from the starting heat source temperature to at least the target heat source temperature at a rate of less than 10 degrees Celsius per minute. In some embodiments, the heat source is heated from the starting heat source temperature to at least the target heat source temperature at a rate of less than 5 degrees Celsius per minute.

In some embodiments, the heat source is at the target heat source temperature or above for at least 15 seconds. In some embodiments, the heat source is at the target heat source temperature or above for at least 30 seconds. In some embodiments, the heat source is at the target heat source temperature or above for at least 1 minute. In some embodiments, the heat source is at the target heat source temperature or above for at least 2 minutes. In some embodiments, the heat source is at the target heat source temperature or above for at least 3 minutes. In some embodiments, the heat source is at the target heat source temperature or above for at least 4 minutes. In some embodiments, the heat source is at the target heat source temperature or above for at least 5 minutes. In some embodiments, the heat source is at the target heat source temperature or above for at least 8 minutes. In some embodiments, the heat source is at the target heat source temperature or above for at least 10 minutes. In some embodiments, the heat source is at the target heat source temperature or above from 2 minutes to 10 minutes. In some embodiments, the heat source is at the target heat source temperature or above from 2 minutes to 16 minutes. In some embodiments, the heat source is at the target heat source temperature or above from 2 minutes to 20 minutes. In some embodiments, the heat source is at the target heat source temperature or above from 5 minutes to 10 minutes. In some embodiments, the heat source is at the target heat source temperature or above from 5 minutes to 16 minutes. In some embodiments, the heat source is at the target heat source temperature or above from 5 minutes to 20 minutes. In some embodiments, the heat source is at the target heat source temperature or above from 8 minutes to 20 minutes.

In some embodiments the sample is washed (once, twice, three times, more than three times, etc.) in between the treatment and the addition of a new PBE.

### Heating of Sample to Target Sample Temperature or Heating Temperature

The method comprises heating the sample with a volume of solution comprising citrate buffer with a pH from 5 to 7, the volume of solution covering the sample at a volume to surface area ration from 5µl/cm² to 500µl/cm², wherein the sample is heated to a target sample temperature (e.g., the substrate on which the sample is disposed is heated to a particular sample target sample temperature, wherein the temperature of the substrate on which the sample is disposed is likely the same as the temperature of the sample) or the sample is heated at a heating sample temperature, e.g., the sample is incubated in the presence of a heating sample temperature, e.g., the heating sample temperature refers to the temperature to which the sample is exposed. The heating sample temperature may not necessarily cause the sample to reach the same temperature, e.g., depending on how long the sample is exposed to the heating sample temperature.

The sample may be heated at the target sample temperature or in the presence of the heating sample temperature for a length of time. The time may be sufficient to reduce the ability of the first PBE to be further detected in the sample (e.g., reduce the presence of the first PBE, reduce the activity and/or detectability of the first PBE). The target sample temperature and the heating sample temperature are below the boiling point of the solution comprising the buffer. In some embodiments, the target sample temperature and/or the heating sample temperature are from 70°C to 100°C, at or above 75°C, at or above 80°C, at or above 90°C, etc.

The length of time that the sample is at the target sample temperature or is heated at the heating sample temperature is from 1 to 20 minutes (e.g., from 1 to 16 minutes, from 2 to 16 minutes, from 4 to 16 minutes, from 5 to 16 minutes, from 6 to 16 minutes, 18 minutes or less, 10 minutes or less, 8 minutes or less, etc. The automated slide stainers used for methods of the present invention may require additional time to heat the slide/sample to the target sample temperature, e.g., the slide/sample may be at the target sample temperature for the above specified time (a holding time), but the heating step of the machine to get the slide/sample to the target sample temperature will be longer than the holding time.

In some embodiments, the sample is raised to a target sample temperature of 90°C over a period of 3 minutes, 4 minutes, 5 minutes, 6 minutes, 7 minutes, 8 minutes, 9 minutes, 10 minutes, more than 10 minutes, etc. As an example, the sample is at 37°C and is raised to 90°C over a period of 8 minutes (the temperature slowly ramps up to 90°C). In some embodiments, the sample is raised to a target sample temperature from 90°C to 99°C over a period of 3 minutes, 4 minutes, 5 minutes, 6 minutes, 7 minutes, 8 minutes, 9 minutes, 10 minutes, more than 10 minutes, etc. In some embodiments, the sample is raised to a target sample temperature of from 80°C to 90°C over a period of 3 minutes, 4 minutes, 5 minutes, 6 minutes, 7 minutes, 8 minutes, 9 minutes, 10 minutes, more than 10 minutes, etc. In some embodiments, the sample is raised to a target sample temperature of from 75°C to 85°C over a period of 3 minutes, 4 minutes, 5 minutes, 6 minutes, 7 minutes, 8 minutes, 9 minutes, 10 minutes, more than 10 minutes, etc. In some embodiments, the sample is raised to a target sample temperature from 75°C to 90°C over a period of 3 minutes, 4 minutes, 5 minutes, 6 minutes, 7 minutes, 8 minutes, 9 minutes, 10 minutes, more than 10 minutes, etc. In some embodiments, the sample is raised to a target sample temperature of from 80°C to 99°C over a period of 3 minutes, 4 minutes, 5 minutes, 6 minutes, 7 minutes, 8 minutes, 9 minutes, 10 minutes, more than 10 minutes, etc.

In some embodiments, the sample is heated at a heating sample temperature (e.g., the sample is exposed to the heating sample temperature) for a period of 3 minutes, 4 minutes, 5 minutes, 6 minutes, 7 minutes, 8 minutes, 9 minutes, 10 minutes, 12 minutes, 13 minutes, 14 minutes, 15 minutes, more than 10 minutes, more than 12 minutes, more than 14 minutes, etc.

In some embodiments, the particular antibody (primary antibody) requires a particular target sample temperature or heating sample temperature, treatment time (holding time at the target sample temperature or heating sample temperature), and/or pH combination for inactivation, e.g., not all antibodies may necessarily require the same target sample temperature or heating sample temperature, pH, and holding time combination for inactivation. Further examples of times and temperatures of treatment are shown in Table 1 below. The present invention is not limited to these examples.

**Table 1**

| **Example** | **Target sample temperature or Heating sample temperature (°C)** | **Treatment Time (min)** | **pH of solution comprising buffer** |
|---|---|---|---|
| A | 80 | 4 | 5.5 |
| B | 85 | 4 | 5.5 |
| c | 90 | 4 | 5.5 |
| D | 95 | 4 | 5.5 |
| E | 98 | 4 | 5.5 |
| F | 100 | 4 | 5.5 |
| G | 80 | 8 | 6.5 |
| H | 85 | 8 | 6.5 |
| I | 90 | 8 | 6.5 |
| J | 95 | 8 | 6.5 |
| K | 98 | 8 | 6.5 |
| L | 100 | 8 | 6.5 |
| M | 80 | 10 | 5.2 |
| N | 85 | 10 | 5.2 |
| O | 90 | 10 | 5.2 |
| P | 95 | 10 | 5.2 |
| Q | 98 | 10 | 5.2 |
| R | 100 | 10 | 5.8 |
| S | 80 | 12 | 5.8 |
| T | 85 | 12 | 5.8 |
| U | 90 | 12 | 5.8 |
| V | 95 | 12 | 5.8 |
| W | 98 | 12 | 5.8 |
| X | 100 | 12 | 5.8 |
| Y | 80 | 16 | 6.1 |
| Z | 85 | 16 | 6.1 |
| AA | 90 | 16 | 6.1 |
| BB | 95 | 16 | 6.1 |
| CC | 98 | 16 | 6.1 |
| DD | 100 | 16 | 6.1 |
| EE | 80 | 18 | 7.1 |
| FF | 85 | 18 | 7.1 |
| GG | 90 | 18 | 7.1 |
| HH | 95 | 18 | 7.1 |
| II | 98 | 18 | 7.1 |
| JJ | 100 | 18 | 7.1 |
| KK | 80 | 20 | 5.5 |
| LL | 85 | 20 | 5.5 |
| MM | 90 | 20 | 5.5 |
| NN | 95 | 20 | 5.5 |
| OO | 98 | 20 | 5.5 |
| PP | 100 | 20 | 5.5 |

In some embodiments, the thermochemical treatment (heat deactivation) methods of the present invention comprise heating the sample to a temperature, e.g., of 98°C (e.g., a nominal temperature of 100°C or actual temperature of 98°C), however the present invention is not limited to these temperatures. For example, in some embodiments, the methods comprise heating the sample to a nominal temperature of 99°C. In some embodiments, the methods comprise heating the sample to a nominal temperature of 98°C. In some embodiments, the methods comprise heating the sample to a nominal temperature of 97°C. In some embodiments, the methods comprise heating the sample to a nominal temperature of 96°C, etc. In some embodiments, the actual temperature is 99°C. In some embodiments, the actual temperature is 98°C. In some embodiments, the actual temperature is 97°C. In some embodiments, the actual temperature is 96°C. In some embodiments, the actual temperature is 95°C, etc.

In some embodiments, the sample is heated to the temperature for 12 minutes. In some embodiments, the sample is heated to the temperature for 11 minutes. In some embodiments, the sample is heated to the temperature for 10 minutes. In some embodiments, the sample is heated to the temperature for 9 minutes. In some embodiments, the sample is heated to the temperature for 8 minutes or less. In some embodiments, the sample is heated to the temperature for 13 minutes. In some embodiments, the sample is heated to the temperature for 14 minutes. In some embodiments, the sample is heated to the temperature for 15 minutes. In some embodiments, the sample is heated to the temperature for 16 minutes. In some embodiments, the sample is heated to the temperature for 17 minutes. In some embodiments, the sample is heated to the temperature for 18 minutes or more than 18 minutes.

In some embodiments, the heating time represents the time the machine begins to ramp up to the target sample temperature as well as the amount of time that the sample is at the temperature. In some embodiments, the heating time represents the amount of time that the sample is at the temperature. In some embodiments, the heating time represents the time the machine begins to ramp up to the target sample temperature, the amount of time that the sample is at the temperature, and an amount of time the sample begins to cool as the sample gets ready for the next step of the assay.

### Proteinaceous Binding Entities (PBEs)

The method comprises repeating the steps with a second PBE (and optionally a third PBE, a fourth PBE, a fifth PBE, etc.) and repeating the treatment steps in between incubations with a PBE. The treatment need not be performed after the last PBE incubation. Each detectable moiety is capable of being detected (e.g., visualized) in the sample at the location that corresponds to the PBE's target location. The method should maintain the quality of the sample such that the sample morphology remains acceptable as determined by a trained reader.

In some embodiments, the method comprises incubating the sample with a first primary antibody and incubating the sample with a first secondary antibody specific for the first primary antibody in a manner that deposits a detectable moiety at a site at which the first primary antibody binds to a target; treating the sample to reduce the ability of the primary antibody to be further detected in the sample as described above, repeating the incubation with at least a second primary antibody and a second secondary antibody specific for the second primary antibody; and repeating the treatment in between each repeat of the incubation with primary/secondary antibodies.

In some embodiments, the first primary antibody and the second primary antibody each comprise a tag (e.g., a hapten, epitope tag, etc.). In some embodiments, the tag (e.g., a hapten, epitope tag, etc.) of the first primary antibody and the tag (e.g., a hapten, epitope tag, etc.) of the second primary antibody are the same. In some embodiments, the tag (e.g., a hapten, epitope tag, etc.) of the first primary antibody and the tag (e.g., a hapten, epitope tag, etc.) of the second primary antibody are different. In some embodiments, the second secondary antibody is the same as the first secondary antibody, but the secondary antibodies have different detectable labels.

The method of detecting at least two targets (e.g., two, three, four, five, etc.) in the same sample on a single solid substrate may comprise incubating the sample with a first exogenous proteinaceous binding entity (PBE) in a manner that deposits a detectable moiety at a site at which the PBE binds to the PBE's target. For example, this may comprise incubating the sample with a first primary antibody and a first secondary antibody specific for the first primary antibody (e.g., a secondary antibody specific for a tag (e.g., a hapten, epitope tag, etc.) on the first primary antibody, a secondary antibody specific for the species of the primary antibody, etc.). The secondary antibody may be labeled, e.g., with a detectable moiety. The detectable moiety may be deposited at the site at which the PBE binds to its target (e.g., the target to which the primary antibody binds). In some embodiments, the sample is incubated with a primary antibody, a secondary antibody (e.g., a secondary antibody with a tag (e.g., a hapten, epitope tag, etc.)) specific for the primary antibody (e.g., the secondary antibody may be anti-species, anti-hapten, etc.), and a tertiary antibody (e.g., labeled) specific for the secondary antibody (e.g., the tertiary antibody may be anti-tag (e.g., anti-hapten), etc.).

As previously discussed, the first PBE may comprise a first primary antibody. The sample may be incubated with a secondary antibody (e.g., with a label) specific for the first antibody and the label is deposited onto the sample (at the location of the primary antibody, e.g., the target site). Further, the second PBE (and optionally subsequent PBEs) may also comprise primary antibodies and the sample may be incubated with secondary antibodies (e.g., labeled) specific for those primary antibodies. In some embodiments, the primary antibodies each comprise a tag (e.g., a hapten, epitope tag, etc.) (and the secondary antibodies are specific for the respective tags). In some embodiments, the secondary antibodies are anti-species antibodies and comprise a tag (e.g., a hapten, epitope tag, etc.), and a tertiary antibody that is labeled is used to deposit the detectable moiety onto the sample. In some embodiments, the tag (e.g., a hapten, epitope tag, etc.) of a first primary antibody and the tag (e.g., a hapten, epitope tag, etc.) of a second primary antibody are the same. In some embodiments, the tags (e.g., haptens, epitope tags, etc.) of secondary antibodies are the same. In some embodiments, the tag (e.g., a hapten, epitope tag, etc.) of a first primary antibody and the tag (e.g., a hapten, epitope tag, etc.) of a second primary antibody are different. In some embodiments, the tags (e.g., haptens, epitope tags, etc.) of the secondary antibodies are different. In some embodiments, a first secondary antibody is the same as a second secondary antibody except the secondary antibodies have different detectable labels. In some embodiments tertiary antibodies are the same (e.g., anti-tag, e.g., anti-hapten, e.g., directed to the same tag/hapten) but differ in detectable labels.

### Automated Systems

The methods of the present invention are automated. In some embodiments, the methods of the present invention are performed in a closed system (e.g., an automated system), wherein the methods are free from manual steps. For example, as used herein, the term "closed system" may refer to a preprogrammed automated method. The term "closed system" may refer to a method wherein each step is automated, e.g., each step is performed without interruption with a manual step. For example, the methods may be performed in a closed system, and the thermochemical treatment step is integrated into the closed system (the thermochemical treatment is not a manual step).

The present invention also features systems programmed to perform the methods of the present invention. Also described is an automated slide stainer not being part of the invention, the automated slide stainer programmed to perform the disclosed methods. In some embodiments, the automated slide stainer comprises a processor and a memory coupled to the processor. The memory stores computer-readable instructions that, when executed by the processor, cause the processor to perform various operations. Such operations may include but are not limited to: instructing the slide stainer to incubate or contact the sample with at least a first exogenous PBE (e.g., primary antibody, primary antibody and labeled secondary antibody) in a manner resulting in deposition of the PBE in proximity to its target; instructing the slide stainer to contact the sample with reagents in a manner resulting in specific deposition of a detectable moiety in proximity to the PBE; instructing the slide stainer to contact the sample with a volume of solution comprising a citrate buffer, the solution comprising the citrate buffer is at a starting buffer temperature which is below the boiling point of the solution comprising the citrate buffer; instructing the slide stainer to contact the solid substrate with a heat source; and instructing the slide stainer to heat the heat source from a starting heat source temperature to at least a target heat source temperature over a length of time, the solution comprising the citrate buffer is raised from the starting buffer temperature to a second buffer temperature, wherein the target heat source temperature and the second buffer temperature are below the boiling point of the solution comprising the citrate buffer. In some embodiments, the operations include: instructing the slide stainer to repeat the process with a second PBE or additional PBEs. In some embodiments, the operations include: instructing the slide stainer to wash the sample after the thermochemical treatment (e.g., before application of a subsequent PBE).

In some embodiments, the slide stainer comprises at least a first dispenser for dispensing the first PBE onto the sample. In some embodiments, the slide stainer comprises at least a second dispenser for dispensing the second PBE onto the sample. In some embodiments, the slide stainer comprises a plurality of antibody dispensers. In some embodiments, the slide stainer further comprises a dispenser for dispensing the solution comprising the buffer onto the sample. In some embodiments, the slide stainer comprises a heat source (e.g., heat pad). In some embodiments, the slide stainer comprises a dispenser for dispensing a wash buffer onto the sample.

The present invention also features a system comprising a buffer reservoir adapted to dispense a solution comprising a buffer onto a sample disposed on a slide; a heat source; at least one antibody dispenser (e.g., a first antibody dispenser for the first primary antibody, a second antibody dispenser for the second secondary antibody, a plurality of antibody dispensers, etc.) adapted to contact the sample with at least a first primary antibody and a second primary antibody; and a secondary antibody capable of binding to both the first primary antibody and the second primary antibody; and a control module adapted to instruct components (e.g., the buffer reservoir, the heat source, the antibody dispenser, etc.) to perform a method according to claim 1. The antibodies may be pre-diluted.

The present description also features compositions not being part of the invention such as compositions comprising a labeled tissue sample obtained using methods of the present invention. The present description also features compositions comprising a slide with a tissue sample, wherein the tissue sample comprises a primary antibody, a secondary antibody, and a label, wherein the tissue sample is incubated at a target sample temperature or heating sample temperature (e.g., below the boiling point of the solution comprising the buffer) in the presence of a solution comprising a buffer having a pH of 5 or above.

The present invention also features automated methods of multiplex labeling. In some embodiments, the methods comprise heating a slide containing a cell sample labeled with a first primary antibody and a secondary antibody comprising a first detectable label using a thermochemical treatment as described herein. The heat-killed cell sample is then labeled with a second primary antibody and a second secondary antibody comprising a second detectable label. In some embodiments, the secondary antibodies differ only in the identity of the detectable label.

The present invention also features automated methods for multiplex labeling of a single cell sample. In some embodiments, the methods comprise a plurality of labeling steps and a heat kill step performed between each labeling step, wherein: each labeling step comprises contacting the cell sample with a set of one or more primary antibodies and detectably labeled secondary antibodies under conditions sufficient for each primary antibody to specifically bind to its target and each secondary antibody to bind to the primary antibody, wherein each secondary antibody is capable of binding to only a single primary antibody of the set; and each heat kill step is as described herein.

In some embodiments, at least two of the sets of primary antibodies and detectably labeled secondary antibodies comprise the same secondary antibody containing a different detectable label. In some embodiments, the same secondary antibody is a species-specific secondary antibody. In some embodiments, the same secondary antibody is an isotype-specific antibody. In some embodiments, the same secondary antibody is an anti-tag antibody (e.g., anti-hapten antibody, anti-epitope tag antibody, etc.).

The present invention also features a method of eliminating a primary/secondary antibody pair in a fixed cellular sample. In some embodiments, the method comprising a thermochemical treatment method according to the present invention.

Without wishing to limit the present invention to any theory or mechanism, it is believed that the methods and systems of the present invention may be beneficial for automated assays in automated staining devices (e.g., Ventana BenchMark, Dako, Leica Bond, etc.). Such automated devices are already adapted to dispense buffers and reagents and to heat samples to various temperatures (e.g., 37°C - 90°C, 37°C - 95°C, 37°C - 99°C, etc.), whereas such automated devices are not adapted to microwave samples or heat samples to very high temperatures such as 130°C.

As previously discussed, the methods of the present invention are automated, e.g., featured in an automated staining machine. Without wishing to limit the present invention to any theory or mechanism, in some embodiments, the method is free from subjecting the sample to microwaving; in some embodiments, the method is free from formaldehyde vapor treatment; in some embodiments, the method does not cause deterioration in quality of the sample.

Without wishing to limit the present invention to any theory or mechanism, it is believed that the methods of the present invention utilize buffers that do not have high osmolarity, extreme pH, and/or chaotropic agents. Without wising to limit the present invention to any theory or mechanism, it is believed that the methods of the present invention help to maintain tissue morphology and/or help to reduce cross-reactivity if using primary antibodies from the same species. Without wising to limit the present invention to any theory or mechanism, it is believed that the methods of the present invention eliminate the need to choose primary antibody from different species. Without wising to limit the present invention to any theory or mechanism, it is believed that the methods of the present invention allow for immunohistochemistry assays in automated slide staining systems.

The present invention can be further described by the examples below.

### EXAMPLES

The following examples describe non-limiting examples of experiments using methods, compositions, and systems of the present invention.

Example 1: Heat-Kill Step After Ag/1^{st} Ab/2^{nd} Ab complex is formed: Inactivating Ag/1^{st} Ab/2^{nd} Ab complex on tonsil slides. In Example 1, the antigen is CD20, the primary antibody is Rabbit-anti-CD20, and the secondary antibody is goat anti-rabbit-HRP. DAB detection is used (for detecting HRP of secondary antibody). Design: After primary antibody (Rabbit-anti-CD20) binding, Goat anti-Rabbit-HRP is applied, followed by a treatment step according to the present invention (e.g., thermochemical treatment step, "Heat-Kill" step), and DAB detection is used. (The control experiment did not have the heat-kill step before the DAB detection step.) The results are shown in FIG. 2. FIG. 2A shows the control experiment wherein no heat-kill step was used in between application of the secondary antibody and the DAB detection step. FIG. 2B shows that a heat kill step (wherein the heat kill step used a target heat source temperature of 95°C; in this example the heat pad/sample was at 95°C for approximately 2.6 minutes and above 90°C for about 250 seconds) can inactivate the CD20/Rabbit-anti-CD20/Goat anti-Rabbit-HRP complex. Note that in this example, the automated staining machine was programmed to reach a target heat source temperature of 95°C, and the programming parameter for the time setting was 8 minutes. The temperature and time setting in this particular automated staining machine enabled the heat pad/sample to be at 95°C for approximately 2.6 minutes and above 90°C for about 250 seconds. Note that other machines may require different programming parameters to achieve particular target heat source temperatures (and durations), for example to achieve a heat source temperature of 95°C for about 2 to 3 minutes, some machines may need a longer time setting if the heat pad has a slow rate of heating (e.g., the heat pad may need longer to reach the target heat source temperature from the starting heat source temperature). The present invention is not limited to the target heat source temperatures, durations, or programming parameters described herein. A target heat source temperature of 90°C (wherein the heat source was at 90°C for about 2.5 minutes; the machine was programmed to be at 90°C and the machine's time setting was 8 minutes), and a target heat source temperature of 90°C (wherein the heat source was at 90°C for about 10.6 minutes; the machine was programmed to be at 90°C and the machine's time setting was 12 minutes), and a target heat source temperature of 95°C (wherein the heat source was at 95°C for about 9.3 minutes; the machine was programmed to be at 90°C and the machine's time setting was 12 min) can inactivate the CD20/Rabbit-anti-CD20/Goat anti-Rabbit-HRP complex (data not shown). Note this Heat-Kill condition may indicate the loss of HRP enzymatic activity, not necessarily denaturation/elution of CD20/Rabbit-anti-CD20/Goat anti-Rabbit-HRP complex.

Example 2: Heat-Kill Step After Ag/1^{st} Ab complex is formed (before 2^{nd} Ab): Inactivating Ag/1^{st} Ab complex in tonsil slides. In Example 2, the antigen is CD20, and the primary antibody is Rabbit-anti-CD20. Design: Rabbit-anti-CD20 is applied, followed by a Heat-Kill step according to the present invention. Then, a secondary antibody (Goat anti-Rabbit-HRP) is applied followed by a DAB detection step. (The control experiment did not have the heat-kill step after the primary antibody incubation and before the secondary antibody incubation.) The results are shown in FIG. 3. FIG. 1A shows the control experiment that did not use a heat-kill step after the primary antibody incubation and before the secondary antibody incubation. FIG. 3B shows that a heat-kill step (a target heat source temperature of 100°C for 6 min) after the primary antibody application but before the secondary antibody application eliminated CD20 IHC staining. In this example, the heat kill step comprised a target heat source temperature of 97°C. The sample was at the target heat source temperature for about 90 seconds (the sample was also above 90°C for about 2.3 minutes). Note that the machine was actually programmed to 100°C, but the actual temperature achieved was 97°C.

Example 3: Heat-Kill Step After Ag/1^{st} Ab/2^{nd} Ab complex is formed: Determine if 100°C for 12 min can inactivate Ag/1st Ab/2nd Ab complex (e.g., CD20/Rabbit-anti-CD20/Goat anti-Rabbit-HRP) by re-applying Goat anti-Rabbit-HRP after the Heat-Kill step. Design: Test 3 concentrations of 1st Ab CD20 (1.04, 0.52, and 0.26 ug/mL) on tonsil/colon cases. The results are shown in FIG. 4. FIG. 4A shows a control experiment with no heat-kill step (antibody concentration was 1.04 ug/mL). FIG. 4B shows the results from a heat-kill step (target heat source temperature of 97°C) performed after the application of the primary and secondary antibodies but before another application of secondary antibody (and subsequent DAB detection). The heat-kill step in FIG. 4B inactivated Ag/1st Ab/2nd Ab complex as demonstrated by no staining when Goat anti-Rabbit-HRP was reapplied with DAB detection. The results were the same when the other concentrations of antibody were used (0.52 ug/mL or 0.26 ug/mL): the heat-kill step described above inactivated the Ag/1st Ab/2nd Ab complex as no staining was observed when goat anti-rabbit-HRP was reapplied with DAB detection (data not shown).

Example 4.1: Heat-Kill Step in Between Detection of Two Targets (CD20 and FoxP3): Determine if Heat-Kill conditions can inactivate the 1^{st} Ag-CD20/1^{st} Ab-Rabbit anti-CD20 Ab/2^{nd} Ab-Goat anti-Rabbit-HRP complex but allow for detection of FoxP3 (Ag-FoxP3/1^{st} Ab-Rabbit anti-FoxP3 Ab/2^{nd} Ab-Goat anti-Rabbit-HRP/DAB), e.g., by demonstrating FoxP3 signals and not CD20 signals in the 2^{nd} staining cycle. Design: Apply 1^{st} Ab-Rabbit anti-CD20 Ab and 2^{nd} Ab-Goat anti-Rabbit-HRP complex, then perform Heat-Kill step (target heat source temperature of 97 °C), then apply 1^{st} Ab-Rabbit anti-FoxP3 Ab and 2^{nd} Ab-Goat anti-Rabbit-HRP and subsequently perform a detection step (e.g., DAB). The results are shown in FIG. 5. FIG. 5A shows CD20 staining as a control (no heat kill step, no FoxP3 staining). FIG. 5B shows FoxP3 staining as a control (no heat kill step, no CD20 staining). FIG. 5C shows the results of the FoxP3 staining when the heat kill step was used as described above (e.g., after CD20 primary and secondary antibodies were applied but before the FoxP3 primary antibody was applied). FIG. 5C shows that FoxP3 demonstrated comparable staining to the control when the Heat-Kill step was used as described above. The results suggest 1^{st} Ab-Rabbit anti-CD20 Ab/2^{nd} Ab-Goat anti-Rabbit-HRP complex in the 1^{st} staining cycle was successfully inactivated, hence no CD20 staining from the cross-reactivity of *2^{nd} Ab-Goat anti-Rabbit-HRP (2^{nd} cycle)* to *1^{st} Ab-Rabbit anti-CD20 Ab (1^{st} cycle).* The results also suggest that FoxP3 antigen was NOT negatively impacted by the heat-Kill step.

Example 4.2: Heat-Kill Step in Between Detection of Two Targets (CD20 and CD3): Determine if Heat-Kill conditions can inactivate 1^{st} Ag-CD20/1^{st} Ab-Rabbit anti-CD20 Ab/2^{nd} Ab-Goat anti-Rabbit-HRP complex but allow for detection of CD3 (2^{nd} Ag-CD3/1^{st} Ab-Rabbit anti-CD3/2^{nd} Ab-Goat anti-Rabbit-HRP/DAB), e.g., demonstrating CD3 signals and not CD20 signals in the 2^{nd} staining cycle. Design: Apply 1^{st} Ab-Rabbit anti-CD20 Ab and 2^{nd} Ab-Goat anti-Rabbit-HRP complex, then perform a Heat-Kill step (target heat source temperature of 97°C), then apply the 2^{nd} staining cycle reagents (1^{st} Ab-Rabbit anti-CD3 Ab, 2^{nd} Ab-Goat anti-Rabbit-HRP and DAB). Results: like FoxP3 in Example 4.1 and FIG. 5, CD3 demonstrated comparable staining to the control under the Heat-Kill step (data not shown). The results suggest 1^{st} Ab-Rabbit anti-CD20 Ab/2^{nd} Ab-Goat anti-Rabbit-HRP complex in the 1^{st} staining cycle was successfully inactivated, hence no CD20 staining from the cross-reactivity of *2^{nd} Ab-Goat anti-Rabbit-HRP (2^{nd} cycle)* to *1^{st} Ab-Rabbit anti-CD20 Ab (1^{st} cycle).* The results also suggest that CD3 antigen was NOT negatively impacted by the heat-Kill step.

Example 5: Effect of Heat-Kill Step on Antigens (CD3, CD8, CD20, CD68, and FoxP3): Evaluate if the Heat-Kill step has negative impact on the antigens. Design: tested 1 to 5 Heat-Kill cycles on the IHC staining of each of CD3, CD8, CD20, CD68 and FoxP3 target. FIG. 6 shows the results of 5 cycles of a heat-kill step (target heat source temperature of 97°C). FIG. 6A shows control staining of CD3. FIG. 6B shows CD3 staining after 5 cycles of the heat kill step described above. CD3 staining was comparable to that of the control (the Heat-Kill steps did not have negative impact on CD3 staining). FIG. 6C shows control staining of CD8. FIG. 6D shows CD8 staining after 5 cycles of the heat kill step described above. CD8 staining was comparable to that of the control (the Heat-Kill steps did not have negative impact on CD8 staining). FIG. 6E shows control staining of CD68. FIG. 6F shows CD68 staining after 5 cycles of the heat kill step described above. CD68 staining was comparable to that of the control (the Heat-Kill steps did not have negative impact on CD68 staining).

Example 6.1: Effect of Heat-Kill Step on Complex Detection in the Nucleus: Evaluate if Heat-Kill can inactivate Ag-FoxP3/Rabbit-anti-FoxP3/Goat anti-Rabbit-HRP complex in the nucleus (in colon samples). Design: Test Various Heat-Kill conditions: target heat source temperature of 95°C (at 95°C for about 2.5 minutes), target heat source temperature 95°C (at 95°C for about 9.3 minutes), target heat source temperature 100°C (at 100°C for about 90 seconds), and target heat source temperature 100°C (at 100°C for more than 90 seconds). The FoxP3 (rabbit) primary antibody is applied (1ug/mL), then the secondary antibody (Goat anti-rabbit -HRP) is applied followed by a Heat-Kill step. Then, the secondary antibody (Goat anti-Rabbit-HRP) is re-applied followed by detection (DAB). Results (not shown): the following heat kill conditions inactivated the Ag-FoxP3/Rabbit-anti-FoxP3/Goat anti-Rabbit-HRP complex (no FoxP3 staining was observed): target heat source temperature of 95°C (at 95°C for about 2.5 minutes), target heat source temperature 95°C (at 95°C for about 9.3 minutes), target heat source temperature 100°C (at 100°C for about 90 seconds), and target heat source temperature 100°C (at 100°C for more than 90 seconds). This demonstrates that a target heat source temperature of 95°C is sufficient (e.g., for about 2.5 minutes, 9.3 minutes, etc.) as demonstrated no staining after Heat-Kill step. Without wishing to limit the present invention to any theory or mechanism, it is possible that the abundance of the antigen may dictate the harshness of the heat kill condition needed as opposed to the localization of the antigen (e.g., membrane vs. nucleus). For example, a lower abundance antigen (e.g. FoxP3) may not need as harsh heat-Kill conditions as a high abundant antigen (e.g. CD20).

Example 6.2: Effect of Heat-Kill Step on Complex Detection in the Cytoplasm: Evaluate if Heat-Kill can inactivate Ag-CD68/Rabbit-anti-CD68/Goat anti-Rabbit-HRP complex in the cytoplasm (in tonsil or colon sample). Design: Test Various Heat-Kill conditions. The CD68 (rabbit) primary antibody is applied (1ug/mL), then the secondary antibody (Goat anti-rabbit -HRP) is applied followed by a heat-kill step. Then, the secondary antibody (Goat anti-Rabbit-HRP) is reapplied followed by detection (DAB). Results (not shown): the following heat kill conditions inactivated the Ag-CD68/Rabbit-anti-CD68/Goat anti-Rabbit-HRP complex (no CD68 staining was observed): target heat source temperature of 95°C (at 95°C for about 2.5 minutes), target heat source temperature 95°C (at 95°C for about 9.3 minutes), target heat source temperature 100°C (at 100°C for about 90 seconds), and target heat source temperature 100°C (at 100°C for more than 90 seconds). This demonstrates that a target heat source temperature of 95°C is sufficient as demonstrated no staining after Heat-Kill step. Without wishing to limit the present invention to any theory or mechanism, it is possible that the abundance of the antigen may dictate the harshness of the heat kill condition needed as opposed to the localization of the antigen (e.g., membrane vs. nucleus). For example, a lower abundance antigen (e.g. CD68) may not need as harsh heat-Kill conditions as a high abundant antigen (e.g. CD20).

Example 7: Evaluate Heat-Kill with other buffers (e.g., Tris-based Reaction Buffer) has impact on antigens (e.g. membrane markers CD3, CD8 and CD20). Design: Step 1: Apply Heat-Kill step to sample (1 cycle of target heat source temperature of 95°C using Tris-based Reaction Buffer. Step 2: Perform Regular IHC for the markers using 6ug/mL CD3, CD8 or CD20 (rabbit), then Goat anti-rabbit -HRP, and DAB for detection. Results (not shown): 1 cycle of 95°C for 8 min with the Tris-based Reaction Buffer reduced CD3, CD8 and CD20 staining compared to the control.

Example 8: Evaluate Heat-Kill with Citrate Buffer other Markers: To further confirm effectiveness of the heat-kill step using a citrate-based buffer (e.g., CC2 from Ventana Medical Systems, Inc.) on multiple markers on multiple tissue types. Design: Tested CD3 (SP162) on colon, HER2 (4B5) on breast, Keratin 5 on head and neck SCC, Ki-67 on breast, ER on breast, PDL1 (SP263) on head and neck SCC.

**Table 2**

| **Ab** | **SP name** | **cellular location** | **Kd** | **method** | **Note** |
|---|---|---|---|---|---|
| PD-L1 | SP263 | membrane | 1.5 × 10E-11M | Mannual ELISA | |
| ER | SP1 | nucleus | 2 × 10E-10M | Biacore | hybridoma |
| Keratin 5 | SP27 | cytoplasm | 1 × 10E-12M | Biacore | |

The results are shown in FIG. 7. FIG. 7A shows CD3 staining in the colon sample. The top left panel shows control CD3 staining. The top right panel shows a control wherein no primary antibody for CD3 was used. The bottom left panel shows the results when the heat-kill step used a target heat source temperature of 80°C. The bottom right panel shows the results when the heat-kill step used a target heat source temperature of 85°C. Both heat kill conditions were effective. FIG. 7B shows HER2 staining in the breast sample. The top left panel shows control HER2 staining. The top right panel shows a control wherein no primary antibody for HER2 was used. The bottom left panel shows the results when the heat-kill step used a target heat source temperature of 80°C. The bottom right panel shows the results when the heat-kill step used a target heat source temperature of 85°C. Both heat kill conditions were effective. FIG. 7C shows Keratin 5 staining in the head/neck squamous cell carcinoma (HNSCC) sample. The top left panel shows control Keratin 5 staining. The top right panel shows the results when the heat-kill step used a target heat source temperature of 90°C. The bottom left panel shows the results when the heat-kill step used a target heat source temperature of 90°C. The bottom right panel shows the results when the heat-kill step used a target heat source temperature of 95°C. The three heat kill conditions were effective. FIG. 7D shows Ki-67 staining in a breast sample. The top left panel shows control Ki-67 staining. The top right panel shows a control wherein no primary antibody for Ki-67 was used. The bottom left panel shows the results when the heat-kill step used a target heat source temperature of 80°C. The bottom right panel shows the results when the heat-kill step used a target heat source temperature of 85°C. Both heat kill conditions were effective. FIG. 7E shows ER staining in a breast sample. The left panel shows control ER staining. The middle panel shows the results when the heat-kill step used a target heat source temperature of 90°C. The right panel shows the results when the heat-kill step used a target heat source temperature of 95°C. Both heat kill conditions were effective. FIG. 7F shows PDL1 staining in a HNSCC sample. The top left panel shows control PDL1 staining. The top right panel shows the results when the heat-kill step used a target heat source temperature of 80°C. The bottom left panel shows the results when the heat-kill step used a target heat source temperature of 85°C. The bottom right panel shows the results when the heat-kill step used a target heat source temperature of 90°C. The three heat kill conditions were effective.

Example 9: Evaluate Heat-Kill Effectiveness on Fluorophore-Conjugated TSA Detection: Confirm a heat-kill condition (citrate buffer (CC2), target heat source temperature of 97°C) works on fluorophore-conjugated TSA (TSA-fluors) detection (in addition to DAB detection as described above). Design: CD20 (rabbit, membrane marker) primary antibody (1ug/mL) is applied, then the secondary antibody (Goat anti-rabbit -HRP) is applied followed by one cycle of a Heat-Kill step (using citrate buffer (CC2) with a target heat source temperature of 97°C). Then, FoxP3 (rabbit, nuclei marker) primary antibody (1ug/ml) is applied, and then the secondary antibody (Goat anti-Rabbit-HRP) antibody is applied followed by TSA-Cy5 detection. Note the use of the same secondary antibody. FIG. 8 shows the results. FIG. 8A shows control staining of CD20. FIG. 8B shows control FoxP3 staining. FIG. 8C shows FoxP3 staining after the heat-kill step was performed as described above. The heat-kill step described above (citrate buffer (CC2), target heat source temperature of 97°C) inactivated CD20/GaR-HRP complex, as demonstrated by the presence of only nuclei staining (FoxP3). No membrane staining was observed, which would have been indicative of CD20.

Example 10: 5-Plex Multiplex Fluorescent IHC (Using Same-Species Primary Antibodies and Anti-Species Secondary Antibodies). Heat-kill conditions (target heat source temperature of 90°C for 5 min) were applied to a 5-plex (CD3, CD8, CD20, CD68, FoxP3) fluorescent IHC assay (on a tonsil sample) using rabbit primary antibodies for each marker, goat-anti-rabbit-HRP for the secondary antibodies for each marker, and fluorophore-conjugated TSA (TSA-fluors) for detection (TSA-DCC for CD3, TSA-Texas Red for CD8, TSA-FITC for CD20, TSA-R6G for CD68, and TSA-Cy5 for FoxP3). Results (not shown): Initial run of fully automated 5-plex with Same Species Primary Antibodies by heat-kill steps showed working.

Example 11: Evaluation of Heat-Kill Step in Multiplex Assay with PD-L1: NSCLC sample (NSCLC case 150637) was used for multiplex assays using PD-L1 antibody clone SP142 @ 7 µg/ml. PD-L1 antibody was tested at different positions in a multiplex (5-plex) assay (e.g., the 3^{rd} position, 4^{th} position). Table 3 below lists 14 different experiments performed. PD-L1 was in position 3 in experiments 1-3, 7-9, and 13. PD-L1 was in position 4 in experiments 2-6, 10-12, and 14. Note that 90103 (used as the other "antibodies" (Ab1, Ab2, Ab3, Ab4, Ab5)) is the diluent of the PD-L1 antibody (but with no PD-L1 antibody). Effectiveness of the heat-kill process was evaluated with R6G, a fluorescent molecule with high sensitivity and low autofluorescence, and potential subsequent fluorescent molecules cyanine 5 (Cy5) and fluorescein isothiocyanate (FITC). Shown in Table 3, the "detection molecule" used in the 1^{st} detection (Fluor1) was Citrate, and the 2^{nd} detection (Fluor2) and 3^{rd} detection (Fluor3) was tyramide (tyramide signal amplification, TSA). Experiments 1, 4, 7, 10, 13, and 14 used a PD-L1 incubation time of 16 minutes. Experiments 2, 5, 8, and 11 used a PD-L1 incubation time of 24 minutes. Experiments 3, 6, 9, and 12 used a PD-L1 incubation time of 32 minutes.

**Table 3**

| | **PDL1 Inc.** | **Ab1** | **Fluor1** | **Ab2** | **Fluor2** | **Ab3** | **Fluor3** | **Ab4** | **Fluor4** | **Ab5** | **Fluor5** | **Objective** |
|---|---|---|---|---|---|---|---|---|---|---|---|---|
| 1 | 16 | 90103 | Citrate | 90103 | TSA Dil. | PDL1 | TSA Dil. | 90103 | R6G | 90103 | TSA Dil. | PD-L1@3, HD evaluated by R6G |
| 2 | 24 | 90103 | Citrate | 90103 | TSA Dil. | PDL1 | TSA Dil. | 90103 | R6G | 90103 | TSA Dil. | |
| 3 | 32 | 90103 | Citrate | 90103 | TSA Dil. | PDL1 | TSA Dil. | 90103 | R6G | 90103 | TSA Dil. | |
| 4 | 16 | 90103 | Citrate | 90103 | TSA Dil. | 90103 | TSA Dil. | PDL1 | TSA Dil. | 90103 | R6G | PD-L1@4, HD evaluated by R6G |
| 5 | 24 | 90103 | Citrate | 90103 | TSA Dil. | 90103 | TSA Dil. | PDL1 | TSA Dil. | 90103 | R6G | |
| 6 | 32 | 90103 | Citrate | 90103 | TSA Dil. | 90103 | TSA Dil. | PDL1 | TSA Dil. | 90103 | R6G | |
| 7 | 16 | 90103 | Citrate | 90103 | TSA Dil. | PDL1 | TSA Dil. | 90103 | Cy5 | 90103 | FITC | PD-L1@3, HD evaluated by Cy5/FITC |
| 8 | 24 | 90103 | Citrate | 90103 | TSA Dil. | PDL1 | TSA Dil. | 90103 | Cy5 | 90103 | FITC | |
| 9 | 32 | 90103 | Citrate | 90103 | TSA Dil. | PDL1 | TSA Dil. | 90103 | Cy5 | 90103 | FITC | |
| 10 | 16 | 90103 | Citrate | 90103 | TSA Dil. | 90103 | TSA Dil. | PDL1 | TSA Dil. | 90103 | FITC | PD-L1@4, HD evaluated by FITC |
| 11 | 24 | 90103 | Citrate | 90103 | TSA Dil. | 90103 | TSA Dil. | PDL1 | TSA Dil. | 90103 | FITC | |
| 12 | 32 | 90103 | Citrate | 90103 | TSA Dil. | 90103 | TSA Dil. | PDL1 | TSA Dil. | 90103 | FITC | |
| 13 | 16 | 90103 | Citrate | 90103 | TSA Dil. | 90103 | TSA Dil. | 90103 | R6G | 90103 | TSA Dil. | R6G autoFL Ctrl |
| 14 | 16 | 90103 | Citrate | 90103 | TSA Dil. | 90103 | TSA Dil. | 90103 | TSA Dil. | 90103 | R6G | |

FIG. 9 shows the results of Experiment 3 of Table 3. FIG. 9A shows DAPI staining (nuclear staining). FIG. 9B shows autofluorescence (DCC) (100 ms exposure). FIG. 9C shows PD-L1 positive staining (another slide) with R6G (staining shows the outlines of the membranes). FIG. 9D shows the PD-L1 staining using R6G following a heat-kill/heat deactivation step of the present invention (500 ms exposure). Even at a prolonged exposure time of 500 ms, no cross-reactive signal was detected above autofluorescent background level. The results were the same for Experiment 6 and Experiment 9 of Table 1 (data not shown).

Note that the present invention is not limited to the particular PD-L1 antibody used herein. For example, in some embodiments, the PD-L1 antibody is clone SP263. Also, the present invention is not limited to the particular incubations times used herein (e.g., 16 minutes, 24 minutes, 32 minutes). In some embodiments, the antibody is incubated for less than 16 minutes (e.g., 5 minutes, 6 minutes, 7 minutes, 8 minutes, 9 minutes, 10 minutes, 11 minutes, 12 minutes, 13 minutes, 15 minutes). In some embodiments, the antibody is incubated for more than 16 minutes (e.g., 17 minutes, 18 minutes, 19 minutes, 20 minutes, 21 minutes, 22 minutes, 23 minutes, 25 minutes, 26 minutes, 27 minutes, 28 minutes, 29 minutes, 30 minutes, 31 minutes). In some embodiments, the antibody is incubated for more than 32 minutes.

The optimal conditions for the thermochemical treatment (heat deactivation) may depend on several factors. For example, a higher sensitivity detection system (e.g., TSA, FITC) may benefit from the use of more stringent HD conditions. In some embodiments, more stringent HD conditions may be beneficial if a particular marker is highly expressed in a particular tissue or sample. In some embodiments, more stringent HD conditions may be beneficial if the antibody-target complex is very stable. Thus, antibody/marker-specific studies may be useful for determining optimal thermochemical treatment (heat deactivation) conditions.

The scope of the invention is only to be limited by the following claims. Reference numbers recited in the claims are exemplary and for ease of review by the patent office only, and are not limiting in any way. In some embodiments, the figures presented in this patent application are drawn to scale, including the angles, ratios of dimensions, etc. In some embodiments, the figures are representative only and the claims are not limited by the dimensions of the figures. In some embodiments, descriptions of the inventions described herein using the phrase "comprising" includes embodiments that could be described as "consisting of', and as such the written description requirement for claiming one or more embodiments of the present invention using the phrase "consisting of" is met.

The reference numbers recited in the below claims are solely for ease of examination of this patent application, and are exemplary, and are not intended in any way to limit the scope of the claims to the particular features having the corresponding reference numbers in the drawings.

## Claims

1. An automated method for detecting at least two targets in the same sample on a single solid substrate, said method comprising:
a. contacting the sample with at least a first exogenous proteinaceous binding entity (PBE) in a manner resulting in deposition of the first PBE in proximity to its target;
b. contacting the sample with reagents in a manner resulting in specific deposition of a detectable moiety in proximity to the first PBE; and
c. treating the sample to reduce the ability of the first PBE to be further detected in the sample by
contacting the sample with a volume of solution comprising a citrate buffer with a pH from 5 to 7 and contacting the solid substrate with a heat source, the volume of solution covers the sample at a volume to surface area ratio from 5µl/cm² to 500µl/cm², the solution comprising the citrate buffer is at a starting buffer temperature which is below the boiling point of the solution comprising the citrate buffer; and
heating the heat source from a starting heat source temperature to at least a target heat source temperature over a length of time, the solution comprising the citrate buffer is raised from the starting buffer temperature to a second buffer temperature, the target heat source temperature and the second buffer temperature are below the boiling point of the solution comprising the citrate buffer;
d. repeating (a) and (b) with at least a second PBE; and
e. repeating (c) between each repeat of (a) and (b);
wherein after (d), each detectable moiety is capable of being detected in the sample at a location that corresponds to the location of the PBE 's target.

2. The method of Claim 1, wherein heat from the heat source heats the solid substrate and sample, and heat from the solid substrate and sample heats the buffer.

3. The method of Claim 1, wherein the solution comprising the citrate buffer has a pH from 5 to 6.5.

4. The method of Claim 1, wherein the starting buffer temperature is from 18°C to 42°C.

5. The method of Claim 1, wherein the starting heat source temperature is from 18°C to 42°C.

6. The method of Claim 1, wherein the target heat source temperature is at least 80°C.

7. The method of Claim 1, wherein the heat source is heated from the starting heat source temperature to at least the target heat source temperature at a rate from 5 to 15 degrees Celsius per minute.

8. The method of Claim 1, wherein the solution comprising the citrate buffer has a concentration of citrate from 5mM to 20mM or wherein the solution comprising the citrate buffer further comprises a surfactant or wherein the volume of solution is a freestanding puddle.

9. The method of Claim 1, wherein the heat source is at or above the target heat source temperature for at least 15 seconds or wherein the length of time the heat source is heated from the starting heat source temperature to at least the target heat source temperature is at least 3 minutes.

10. The method of Claim 1, wherein the first PBE comprises a first primary antibody and (b) comprises incubating the sample with a first secondary antibody specific for the first primary antibody in a manner resulting in specific deposition of the first secondary antibody in proximity to the first primary antibody; and a second PBE comprises a second primary antibody and (d) comprises incubating the sample with a second secondary antibody specific for the second primary antibody in a manner resulting in specific deposition of the second secondary antibody in proximity to the second primary antibody.

11. The method of Claim 10, wherein the first primary antibody comprises a tag.

12. The method of Claim 1, wherein the method is performed in a closed system.

13. A system comprising:
a. a buffer reservoir adapted to dispense a citrate buffer onto a sample disposed on a slide;
b. a heat source adapted to heat the slide with the sample, the heat source being heated from a starting heat source temperature to a target heat source temperature;
c. at least one antibody dispenser adapted to contact the sample with
i. at least a first primary antibody and a second primary antibody; and
ii. a secondary antibody capable of binding to both the first primary antibody and the second primary antibody; and
d. a control module adapted to instruct (a)-(c) to perform a method according to Claim 1.

14. The system of Claim 13, wherein the system comprises a first antibody dispenser for the first primary antibody and a second antibody dispenser for the second secondary antibody, and a third antibody dispenser for the secondary antibody.

## Patentansprüche

1. Automatisiertes Verfahren zum Detektieren von mindestens zwei Zielen in der gleichen Probe auf einem einzelnen festen Substrat, das Verfahren Folgendes umfassend:
a. Kontaktieren der Probe mit mindestens einer ersten exogenen proteinösen Bindungsentität (PBE) in einer Weise, die zur Ablagerung der ersten PBE in räumlicher Nähe zu ihrem Ziel führt;
b. Kontaktieren der Probe mit Reagenzien in einer Weise, die zur spezifischen Ablagerung eines detektierbaren Rests in räumlicher Nähe zu der ersten PBE führt; und
c. Behandeln der Probe, um die Fähigkeit der ersten PBE zu reduzieren, weiter in der Probe detektiert zu werden, durch
Kontaktieren der Probe mit einem Volumen einer Lösung, die einen Citrat-Puffer mit einem pH-Wert zwischen 5 und 7 enthält, und Kontaktieren des festen Substrats mit einer Wärmequelle, wobei das Volumen der Lösung die Probe mit einem Verhältnis von Volumen zu Oberfläche zwischen 5 µl/cm² und 500 µl/cm² bedeckt, wobei die Lösung, die den Citrat-Puffer enthält, bei einer Pufferanfangstemperatur ist, die unter dem Siedepunkt der Lösung liegt, die den Citrat-Puffer enthält; und
Erwärmen der Wärmequelle von einer Wärmequellenanfangstemperatur auf mindestens eine Wärmequellenzieltemperatur über eine Zeitspanne hinweg, wobei die Lösung, die den Citrat-Puffer enthält, von der Pufferanfangstemperatur auf eine zweite Puffertemperatur erhöht wird, wobei die Wärmequellenzieltemperatur und die zweite Puffertemperatur unter dem Siedepunkt der Lösung liegen, die den Citrat-Puffer enthält;
d. Wiederholen von (a) und (b) mit mindestens einer zweiten PBE; und
e. Wiederholen von (c) zwischen jeder Wiederholung von (a) und (b);
wobei nach (d) jeder detektierbare Rest in der Probe an einem Ort detektiert werden kann, der dem Ort des Ziels der PBE entspricht.

2. Verfahren nach Anspruch 1, wobei Wärme von der Wärmequelle das feste Substrat und die Probe erwärmt und Wärme aus dem festen Substrat und der Probe den Puffer erwärmt.

3. Verfahren nach Anspruch 1, wobei die Lösung, die den Citrat-Puffer enthält, einen pH-Wert zwischen 5 und 6,5 aufweist.

4. Verfahren nach Anspruch 1, wobei die Pufferanfangstemperatur zwischen 18 °C und 42 °C ist.

5. Verfahren nach Anspruch 1, wobei die Wärmequellenanfangstemperatur zwischen 18 °C und 42 °C ist.

6. Verfahren nach Anspruch 1, wobei die Wärmequellenzieltemperatur mindestens 80 °C ist.

7. Verfahren nach Anspruch 1, wobei die Wärmequelle von der Wärmequellenanfangstemperatur mit einer Rate zwischen 5 und 15 Grad Celsius pro Minute auf mindestens die Wärmequellenzieltemperatur erwärmt wird.

8. Verfahren nach Anspruch 1, wobei die Lösung, die den Citrat-Puffer enthält, eine Konzentration von Citrat zwischen 5 mM und 20 mM aufweist oder wobei die Lösung, die den Citrat-Puffer enthält, weiterhin ein Tensid enthält oder wobei das Lösungsvolumen eine freistehende Lache ist.

9. Verfahren nach Anspruch 1, wobei die Wärmequelle für mindestens 15 Sekunden auf oder über der Wärmequellenzieltemperatur ist oder wobei die Zeitspanne, welche die Wärmequelle von der Wärmequellenanfangstemperatur auf mindestens die Wärmequellenzieltemperatur erwärmt wird, mindestens 3 Minuten ist.

10. Verfahren nach Anspruch 1, wobei die erste PBE einen ersten primären Antikörper enthält und (b) Bebrüten der Probe mit einem ersten sekundären Antikörper, der für den ersten primären Antikörper spezifisch ist, in einer Weise umfasst, die zur spezifischen Ablagerung des ersten sekundären Antikörpers in räumlicher Nähe zu dem ersten primären Antikörper führt; und eine zweite PBE einen zweiten primären Antikörper enthält und (d) Bebrüten der Probe mit einem zweiten sekundären Antikörper, der für den zweiten primären Antikörper spezifisch ist, in einer Weise umfasst, die zur spezifischen Ablagerung des zweiten sekundären Antikörpers in räumlicher Nähe zu dem zweiten primären Antikörper führt.

11. Verfahren nach Anspruch 10, wobei der erste primäre Antikörper einen Marker enthält.

12. Verfahren nach Anspruch 1, wobei das Verfahren in einem geschlossenen System durchgeführt wird.

13. System, Folgendes umfassend:
a. ein Pufferreservoir, das eingerichtet ist, um einen Citrat-Puffer auf eine Probe auszugeben, die auf einem Objektträger angeordnet ist;
b. eine Wärmequelle, die eingerichtet ist, um den Objektträger mit der Probe zu erwärmen, wobei die Wärmequelle von einer Wärmequellenanfangstemperatur auf eine Wärmequellenzieltemperatur erwärmt wird;
c. mindestens einen Antikörperspender, der eingerichtet ist, um die Probe zu kontaktieren mit
i. mindestens einem ersten primären Antikörper und einem zweiten primären Antikörper; und
ii. einem sekundären Antikörper, der sich sowohl an den ersten primären Antikörper als auch an den zweiten primären Antikörper binden kann; und
d. ein Steuerungsmodul, das eingerichtet ist, um (a) bis (c) zu instruieren, um ein Verfahren nach Anspruch 1 durchzuführen.

14. System nach Anspruch 13, wobei das System einen ersten Antikörperspender für den ersten primären Antikörper und einen zweiten Antikörperspender für den zweiten sekundären Antikörper und einen dritten Antikörperspender für den sekundären Antikörper umfasst.

## Revendications

1. Procédé automatisé pour la détection d'au moins deux cibles dans le même échantillon sur un unique substrat solide, ledit procédé comprenant :
a. la mise en contact de l'échantillon avec au moins une première entité de liaison protéique (PBE) exogène d'une manière entraînant un dépôt de la première PBE à proximité de sa cible ;
b. la mise en contact de l'échantillon avec des réactifs d'une manière entraînant un dépôt spécifique d'un fragment détectable à proximité de la première PBE ; et
c. le traitement de l'échantillon pour réduire la capacité de la première PBE à être davantage détectée dans l'échantillon par
mise en contact de l'échantillon avec un volume de solution comprenant un tampon citrate doté d'un pH de 5 à 7 et mise en contact du substrat solide avec une source de chaleur, le volume de solution couvrant l'échantillon à raison d'un rapport de volume sur superficie de 5 µl/cm² à 500 µl/cm², la solution comprenant le tampon citrate étant à une température de tampon de départ qui est inférieure au point d'ébullition de la solution comprenant le tampon citrate ; et
chauffage de la source de chaleur depuis une température de source de chaleur de départ jusqu'à au moins une température de source de chaleur cible sur une période de temps, la solution comprenant le tampon citrate étant élevée de la température de tampon de départ jusqu'à une deuxième température de tampon, la température de source de chaleur cible et la deuxième température de tampon étant inférieures au point d'ébullition de la solution comprenant le tampon citrate ;
d. la répétition de (a) et (b) avec au moins une deuxième PBE ; et
e. la répétition de (c) entre chaque répétition de (a) et (b) ;
après (d), chaque fragment détectable étant capable d'être détecté dans l'échantillon au niveau d'un emplacement qui correspond à l'emplacement de la cible de la PBE.

2. Procédé selon la revendication 1, la chaleur provenant de la source de chaleur chauffant le substrat solide et l'échantillon, et la chaleur provenant du substrat solide et de l'échantillon chauffant le tampon.

3. Procédé selon la revendication 1, la solution comprenant le tampon citrate possédant un pH de 5 à 6,5.

4. Procédé selon la revendication 1, la température de tampon de départ étant de 18 °C à 42 °C.

5. Procédé selon la revendication 1, la température de source de chaleur de départ étant de 18 °C à 42 °C.

6. Procédé selon la revendication 1, la température de source de chaleur cible étant d'au moins 80 °C.

7. Procédé selon la revendication 1, la source de chaleur étant chauffée de la température de source de chaleur de départ jusqu'à au moins la température de source de chaleur cible à une vitesse de 5 à 15 degrés Celsius par minute.

8. Procédé selon la revendication 1, la solution comprenant le tampon citrate possédant une concentration de citrate de 5 mM à 20 mM ou la solution comprenant le tampon citrate comprenant en outre un tensioactif ou le volume de solution étant une flaque indépendante.

9. Procédé selon la revendication 1, la source de chaleur étant à la température de source de chaleur cible ou au-dessus pendant au moins 15 secondes ou la période de temps où la source de chaleur est chauffée depuis la température de source de chaleur de départ jusqu'à au moins la température de source de chaleur cible étant d'au moins 3 minutes.

10. Procédé selon la revendication 1, la première PBE comprenant un premier anticorps primaire et (b) comprenant une incubation de l'échantillon avec un premier anticorps secondaire spécifique pour le premier anticorps primaire d'une manière entraînant un dépôt spécifique du premier anticorps secondaire à proximité du premier anticorps primaire ; et une deuxième PBE comprenant un deuxième anticorps primaire et (d) comprenant une incubation de l'échantillon avec un deuxième anticorps secondaire spécifique pour le deuxième anticorps primaire d'une manière entraînant un dépôt spécifique du deuxième anticorps secondaire à proximité du deuxième anticorps primaire.

11. Procédé selon la revendication 10, le premier anticorps primaire comprenant une étiquette.

12. Procédé selon la revendication 1, le procédé étant réalisé dans un système clos.

13. Système comprenant :
a. un réservoir de tampon conçu pour distribuer un tampon citrate sur un échantillon disposé sur une lame ;
b. une source de chaleur conçue pour chauffer la lame avec l'échantillon, la source de chaleur étant chauffée depuis une température de source de chaleur de départ jusqu'à une température de source de chaleur cible ;
c. au moins un distributeur d'anticorps conçu pour mettre en contact l'échantillon avec
i. au moins un premier anticorps primaire et un deuxième anticorps primaire ; et
ii. un anticorps secondaire capable de se lier à la fois au premier anticorps primaire et au deuxième anticorps primaire ; et
d. un module de commande conçu pour ordonner à (a)-(c) de réaliser un procédé selon la revendication 1.

14. Système selon la revendication 13, le système comprenant un premier distributeur d'anticorps pour le premier anticorps primaire et un deuxième distributeur d'anticorps pour le deuxième anticorps secondaire, et un troisième distributeur d'anticorps pour l'anticorps secondaire.
